# EUROPEAN PATENT SPECIFICATION

(11) **EP 2 834 354 B1**
(45) Date of publication and mention of the grant of the patent: **16.08.2017**
(21) Application number: 13713899.6
(22) Date of filing: 03.04.2013
(51) Int. Cl.: C12N 11/04, C12P 19/02, C12N 11/10, C12P 19/24, C12N 11/12, C12N 9/90

(54) **IMPROVED GALACTOSE ISOMERASES AND USE THEREOF IN THE PRODUCTION OF TAGATOSE**
VERBESSERTE GALACTOSEISOMERASEN UND VERWENDUNG DAVON BEI DER HERSTELLUNG VON TAGATOSE
GALACTOSE ISOMÉRASES AMÉLIORÉES ET UTILISATION DE CELLES-CI DANS LA PRODUCTION DE TAGATOSE

(30) Priority: 04.04.2012 EP 12163188; 04.04.2012 US 201261620002 P
(43) Date of publication of application: 11.02.2015
(73) Proprietor: Nutrilab N.V., 3550 Heusden-Zolder (BE)
(72) Inventor: FRANÇOIS, Jean Marie, 4557 Soheit-Tinlot (BE); COUNSON, Melody Jean-Pierre Maryline, 6690 Vielsalm (BE); BOULEAU, Cindy Amandine Nadège Thérèse, 4100 Seraing (BE); BRANS, Alain Fernand Francis, 4052 Beaufays (BE); DELMARCELLE, Michaël André J., 1457 Walhain (BE); HENDRICKX, Wilfried Gerard Jules, 3593 Paal Beringen (BE); MIRZAIAN, Hamik, 04100 Latina (IT); FORTUNATO, Antonio, 04100 Latina (IT); FREICHELS, Régine, 4031 Angleur (BE); VASTENAVOND, Christian Manuel, 3272 Testelt (Scherpenheuvel-Zichem) (BE)
(74) Representative: Nederlandsch Octrooibureau
(86) International application number: PCT/EP2013/057019
(87) International publication number: WO 2013/150069

(56) References cited:
- WO-A1-2009/066127
- WO-A2-03/008593
- DEOK-KUN OH: "Tagatose: properties, applications, and biotechnological processes", APPLIED MICROBIOLOGY AND BIOTECHNOLOGY, SPRINGER, BERLIN, DE, vol. 76, no. 1, 10 May 2007 (2007-05-10), pages 1-8, XP019538798, ISSN: 1432-0614, DOI: 10.1007/S00253-007-0981-1
- RHIMI M ET AL: "Production of d-tagatose, a low caloric sweetener during milk fermentation using l-arabinose isomerase", BIORESOURCE TECHNOLOGY, ELSEVIER BV, GB, vol. 102, no. 3, 1 February 2011 (2011-02-01), pages 3309-3315, XP027582991, ISSN: 0960-8524 [retrieved on 2011-01-04]

### SEQUENCE LISTING

<110> Nutrilab N.V.
<120> Improved galactose isomerases and use thereof in the production of tagatose
<130> P6036885PCT
<150> US 61/620,002
   <151> 2012-04-04
<150> EP 12163188.1
   <151> 2012-04-04
<160> 8
<170> PatentIn version 3.3
<210> 1
   <211> 496
   <212> PRT
   <213> Geobacilus stearothermophilus
<400> 1
   Met Leu Ser Leu Arg Pro Tyr Glu Phe Trp Phe Val Thr Gly Ser Gln
   1               5                   10                  15
   His Leu Tyr Gly Glu Glu Ala Leu Lys Gln Val Glu Glu His Ser Met
               20                  25                  30
   Met Ile Val Asn Glu Leu Asn Gln Asp Ser Val Phe Pro Phe Pro Leu
           35                  40                  45
   Val Phe Lys Ser Val Val Thr Thr Pro Glu Glu Ile Arg Arg Val Cys
       50                  55                  60
   Leu Glu Ala Asn Ala Ser Glu Gln Cys Ala Gly Val Ile Thr Trp Met
   65                  70                  75                  80
   His Thr Phe Ser Pro Ala Lys Met Trp Ile Gly Gly Leu Leu Glu Leu
                   85                  90                  95
   Arg Lys Pro Leu Leu His Leu His Thr Gln Phe Asn Arg Asp Ile Pro
               100                 105                 110
   Trp Asp Ser Ile Asp Met Asp Phe Met Asn Leu Asn Gln Ser Ala His
           115                 120                 125
   Gly Asp Arg Glu Tyr Gly Phe Ile Gly Ala Arg Met Gly Val Ala Arg
       130                 135                 140
   Lys Val Val Val Gly His Trp Glu Asp Pro Glu Val Arg Glu Arg Leu
   145 150 155 160
   Ala Lys Trp Met Arg Thr Ala Val Ala Phe Ala Glu Ser Arg Asn Leu
   165 170 175
   Lys Val Ala Arg Phe Gly Asp Asn Met Arg Glu Val Ala Val Thr Glu
   180 185 190
   Gly Asp Lys Val Gly Ala Gln Ile Gln Phe Gly Trp Ser Val Asn Gly
   195 200 205
   Tyr Gly Ile Gly Asp Leu Val Gln Tyr Ile Arg Asp Val Ser Glu Gln
   210 215 220
   Lys Val Asn Glu Leu Leu Asp Glu Tyr Glu Glu Leu Tyr Asp Ile Val
   225 230 235 240
   Pro Ala Gly Arg Gln Glu Gly Pro Val Arg Glu Ser Ile Arg Glu Gln
   245 250 255
   Ala Arg Ile Glu Leu Gly Leu Lys Ala Phe Leu Gln Asp Gly Asn Phe
   260 265 270
   Thr Ala Phe Thr Thr Thr Phe Glu Asp Leu His Gly Met Lys Gln Leu
   275 280 285
   Pro Gly Leu Ala Val Gln Arg Leu Met Ala Glu Gly Tyr Gly Phe Gly
   290 295 300
   Gly Glu Gly Asp Trp Lys Thr Ala Ala Leu Val Arg Leu Met Lys Val
   305 310 315 320
   Met Ala Asp Gly Lys Gly Thr Ser Phe Met Glu Asp Tyr Thr Tyr His
   325 330 335
   Leu Glu Pro Gly Asn Glu Met Ile Leu Gly Ala His Met Leu Glu Val
   340 345 350
   Cys Pro Thr Ile Ala Ala Thr Arg Pro Arg Ile Glu Val His Pro Leu
   355 360 365
   Ser Ile Gly Gly Lys Glu Asp Pro Ala Arg Leu Val Phe Asp Gly Gly
   370 375 380
   Glu Gly Ala Ala Val Asn Ala Ser Leu Ile Asp Leu Gly His Arg Phe
   385 390 395 400
   Arg Leu Ile Val Asn Glu Val Asp Ala Val Lys Pro Glu His Asp Met
   405 410 415
   Pro Lys Leu Pro Val Ala Arg Ile Leu Trp Lys Pro Arg Pro Ser Leu
   420 425 430
   Arg Asp Ser Ala Glu Ala Trp Ile Leu Ala Gly Gly Ala His His Thr
   435 440 445
   Cys Phe Ser Phe Ala Val Thr Thr Glu Gln Leu Gln Asp Phe Ala Glu
   450 455 460
   Met Ala Gly Ile Glu Cys Val Val Ile Asn Glu His Thr Ser Val Ser
   465 470 475 480
   Ser Phe Lys Asn Glu Leu Lys Trp Asn Glu Val Phe Trp Arg Gly Arg
   485 490 495
<210> 2
   <211> 496
   <212> PRT
   <213> Artificial
<220>
   <223> mutated L-arabinose isomerase from G. stearothermophilus
<400> 2
   Met Leu Ser Leu Arg Pro Tyr Glu Phe Trp Phe Val Thr Gly Ser Gln
   1               5                   10                  15
   His Leu Tyr Gly Glu Glu Ala Leu Lys Gln Val Glu Glu His Ser Met
               20                  25                  30
   Met Ile Val Asn Glu Leu Asn Gln Asp Ser Val Phe Pro Phe Pro Leu
           35                  40                  45
   Val Phe Lys Ser Val Val Thr Thr Pro Glu Glu Ile Arg Arg Val Cys
       50                  55                  60
   Leu Glu Ala Asn Ala Ser Glu Gln Cys Ala Gly Val Ile Thr Trp Met
   65                  70                  75                  80
   His Thr Phe Ser Pro Ala Lys Met Trp Ile Gly Gly Leu Leu Glu Leu
                   85                  90                  95
   Arg Lys Pro Leu Leu His Leu His Thr Gln Phe Asn Arg Asp Ile Pro
               100                 105                 110
   Trp Asp Ser Ile Asp Met Asp Phe Met Asn Leu Asn Gln Ser Ala His
   115 120 125
   Gly Asp Arg Glu Tyr Gly Phe Ile Gly Ala Arg Met Gly Val Ala Arg
   130 135 140
   Lys Val Val Val Gly His Trp Glu Asp Pro Glu Val Arg Glu Arg Leu
   145 150 155 160
   Ala Lys Trp Met Arg Thr Ala Val Ala Phe Ala Glu Ser Arg Asn Leu
   165 170 175
   Lys Val Ala Arg Phe Gly Asp Asn Met Arg Glu Val Ala Val Thr Glu
   180 185 190
   Gly Asp Lys Val Gly Ala Gln Ile Gln Phe Gly Trp Ser Val Asn Gly
   195 200 205
   Tyr Gly Ile Gly Asp Leu Val Gln Tyr Ile Arg Asp Val Ser Glu Gln
   210 215 220
   Lys Val Asn Glu Leu Leu Asp Glu Tyr Glu Glu Leu Tyr Asp Ile Val
   225 230 235 240
   Pro Ala Gly Arg Gln Glu Gly Pro Val Arg Glu Ser Ile Arg Glu Gln
   245 250 255
   Ala Arg Ile Glu Leu Gly Leu Lys Ala Phe Leu Gln Asp Gly Asn Phe
   260 265 270
   Thr Ala Phe Thr Thr Thr Phe Glu Asp Leu His Gly Met Lys Gln Leu
   275 280 285
   Pro Gly Leu Ala Val Gln Arg Leu Met Ala Glu Gly Tyr Gly Phe Gly
   290 295 300
   Gly Glu Gly Asp Trp Lys Thr Ala Ala Leu Val Arg Leu Met Lys Val
   305 310 315 320
   Met Ala Asp Gly Lys Gly Thr Ser Phe Met Glu Asp Tyr Thr Tyr His
   325 330 335
   Leu Glu Pro Gly Asn Glu Leu Ile Leu Gly Ala His Met Leu Glu Val
   340 345 350
   Asp Pro Thr Ile Ala Ala Thr Arg Pro Arg Ile Glu Val His Pro Leu
   355 360 365
   Ser Ile Gly Gly Lys Glu Asp Pro Val Arg Leu Val Phe Asp Gly Gly
       370                 375                 380
   Glu Gly Ala Ala Val Asn Ala Ser Leu Ile Asp Leu Gly His Arg Phe
   385                 390                 395                 400
   Arg Leu Ile Val Asn Glu Val Asp Ala Val Lys Pro Glu His Asp Leu
                   405                 410                 415
   Pro Lys Leu Pro Val Ala Arg Ile Leu Trp Lys Pro Arg Pro Ser Phe
               420                 425                 430
   Arg Asp Ser Ala Glu Ala Trp Ile Leu Ala Gly Gly Ala His His Thr
           435                 440                 445
   Val Phe Ser Phe Ala Val Thr Thr Glu Gln Leu Gln Asp Phe Ala Glu
       450                 455                 460
   Ile Ala Gly Ile Glu Leu Val Val Ile Asn Glu His Thr Ser Val Ser
   465                 470                 475                 480
   Ser Phe Lys Asn Glu Leu Lys Trp Asn Glu Val Phe Trp Arg Gly Arg
                   485                 490                 495
<210> 3
   <211> 495
   <212> PRT
   <213> Artificial
<220>
   <223> mutated L-arabinose isomerase from G. stearothermophilus
<400> 3
   Met Ser Leu Arg Pro Tyr Glu Phe Trp Phe Val Thr Gly Ser Gln His
   1               5                   10                  15
   Leu Tyr Gly Glu Glu Ala Leu Lys Gln Val Glu Glu His Ser Ser Lys
               20                  25                  30
   Ile Val Asn Glu Leu Asn Gln Asp Ser Val Phe Pro Phe Pro Leu Val
           35                  40                  45
   Phe Lys Ser Val Val Thr Thr Pro Glu Glu Ile Arg Arg Val Phe Leu
       50                  55                  60
   Glu Ala Asn Ala Ser Glu Gln Cys Ala Gly Val Ile Thr Trp Leu His
   65                  70                  75                  80
   Thr Phe Ser Pro Ala Lys Ser Trp Ile Gly Gly Leu Leu Glu Leu Arg
   85 90 95
   Lys Pro Leu Leu His Leu His Thr Gln Phe Asn Arg Asp Ile Pro Trp
   100 105 110
   Asp Ser Ile Asp Met Asp Phe Met Asn Leu Asn Gln Ser Ala His Gly
   115 120 125
   Asp Arg Glu Tyr Gly Phe Ile Gly Ala Arg Leu Gly Val Ala Arg Lys
   130 135 140
   Val Val Val Gly His Trp Glu Asp Pro Glu Val Arg Glu Arg Leu Ala
   145 150 155 160
   Lys Trp Met Arg Thr Ala Val Ala Phe Ala Glu Ser Arg Asn Leu Lys
   165 170 175
   Val Ala Arg Phe Gly Asp Asn Met Arg Glu Val Ala Val Thr Glu Gly
   180 185 190
   Asp Lys Val Gly Ala Gln Ile Gln Phe Gly Trp Ser Val Asn Thr Tyr
   195 200 205
   Gly Ile Gly Asp Leu Val Gln Tyr Ile Arg Asp Val Ser Glu Gln Lys
   210 215 220
   Val Asn Glu Leu Leu Asp Glu Tyr Glu Glu Leu Tyr Asp Ile Val Pro
   225 230 235 240
   Ala Gly Arg Gln Glu Gly Pro Val Arg Glu Ser Ile Arg Glu Gln Ala
   245 250 255
   Arg Ile Glu Leu Gly Leu Lys Ala Phe Leu Gln Asp Gly Asn Phe Thr
   260 265 270
   Ala Phe Thr Thr Thr Phe Glu Asp Leu His Gly Leu Lys Gln Leu Pro
   275 280 285
   Gly Leu Ala Val Gln Arg Leu Leu Ala Glu Gly Tyr Gly Phe Gly Gly
   290 295 300
   Glu Gly Asp Trp Lys Thr Ala Ala Leu Val Arg Leu Leu Lys Val Leu
   305 310 315 320
   Ala Asp Gly Lys Gly Thr Ser Phe Met Glu Asp Tyr Thr Tyr His Leu
   325 330 335
   Glu Pro Gly Asn Glu Met Ile Leu Gly Ala His Met Leu Glu Val Cys
               340                 345                 350
   Pro Thr Ile Ala Ala Thr Arg Pro Arg Ile Glu Val His Pro Leu Ser
           355                 360                 365
   Ile Gly Gly Lys Glu Asp Pro Ala Arg Leu Val Phe Asp Gly Gly Glu
       370                 375                 380
   Gly Ala Ala Val Asn Ala Ser Leu Ile Asp Leu Gly His Arg Phe Arg
   385                 390                 395                 400
   Leu Ile Val Asn Glu Val Asp Ala Val Lys Pro Glu His Asp Met Pro
                   405                 410                 415
   Lys Leu Pro Val Ala Arg Ile Leu Trp Lys Pro Arg Pro Ser Leu Arg
               420                 425                 430
   Asp Ser Ala Glu Ala Trp Ile Leu Ala Gly Gly Ala His His Thr Cys
           435                 440                 445
   Phe Ser Phe Ala Val Thr Thr Glu Gln Leu Gln Asp Phe Ala Glu Met
       450                 455                 460
   Ala Gly Ile Glu Cys Val Val Ile Asn Glu His Thr Ser Val Ser Ser
   465                 470                 475                 480
   Phe Lys Asn Glu Leu Lys Trp Asn Glu Val Phe Trp Arg Gly Arg
                   485                 490                 495
<210> 4
   <211> 495
   <212> PRT
   <213> Artificial
<220>
   <223> mutated L-arabinose isomerase from G. stearothermophilus
<400> 4
   Met Ser Leu Arg Pro Tyr Glu Phe Trp Phe Val Thr Gly Ser Gln His
   1               5                   10                  15
   Leu Tyr Gly Glu Glu Ala Leu Lys Gln Val Glu Glu His Ser Ser Lys
               20                  25                  30
   Ile Val Asn Glu Leu Asn Gln Asp Ser Val Phe Pro Phe Pro Leu Val
           35                  40                  45
   Phe Lys Ser Val Val Thr Thr Pro Glu Glu Ile Arg Arg Val Phe Leu
   50 55 60
   Glu Ala Asn Ala Ser Glu Gln Cys Ala Gly Val Ile Thr Trp Leu His
   65 70 75 80
   Thr Phe Ser Pro Ala Lys Ser Trp Ile Gly Gly Leu Leu Glu Leu Arg
   85 90 95
   Lys Pro Leu Leu His Leu His Thr Gln Phe Asn Arg Asp Ile Pro Trp
   100 105 110
   Asp Ser Ile Asp Met Asp Phe Met Asn Leu Asn Gln Ser Ala His Gly
   115 120 125
   Asp Arg Glu Tyr Gly Phe Ile Gly Ala Arg Leu Gly Val Ala Arg Lys
   130 135 140
   Val Val Val Gly His Trp Glu Asp Pro Glu Val Arg Glu Arg Leu Ala
   145 150 155 160
   Lys Trp Met Arg Thr Ala Val Ala Phe Ala Glu Ser Arg Asn Leu Lys
   165 170 175
   Val Ala Arg Phe Gly Asp Asn Met Arg Glu Val Ala Val Thr Glu Gly
   180 185 190
   Asp Lys Val Gly Ala Gln Ile Gln Phe Gly Trp Ser Val Asn Thr Tyr
   195 200 205
   Gly Ile Gly Asp Leu Val Gln Tyr Ile Arg Asp Val Ser Glu Gln Lys
   210 215 220
   Val Asn Glu Leu Leu Asp Glu Tyr Glu Glu Leu Tyr Asp Ile Val Pro
   225 230 235 240
   Ala Gly Arg Gln Glu Gly Pro Val Arg Glu Ser Ile Arg Glu Gln Ala
   245 250 255
   Arg Ile Glu Leu Gly Leu Lys Ala Phe Leu Gln Asp Gly Asn Phe Thr
   260 265 270
   Ala Phe Thr Thr Thr Phe Glu Asp Leu His Gly Met Lys Gln Leu Pro
   275 280 285
   Gly Leu Ala Val Gln Arg Leu Met Ala Glu Gly Tyr Gly Phe Gly Gly
   290 295 300
   Glu Gly Asp Trp Lys Thr Ala Ala Leu Val Arg Leu Met Lys Val Met
   305                 310                 315                 320
   Ala Asp Gly Lys Gly Thr Ser Phe Met Glu Asp Tyr Thr Tyr His Leu
                   325                 330                 335
   Glu Pro Gly Asn Glu Met Ile Leu Gly Ala His Met Leu Glu Val Cys
               340                 345                 350
   Pro Thr Ile Ala Ala Thr Arg Pro Arg Ile Glu Val His Pro Leu Ser
           355                 360                 365
   Ile Gly Gly Lys Glu Asp Pro Ala Arg Leu Val Phe Asp Gly Gly Glu
       370                 375                 380
   Gly Ala Ala Val Asn Ala Ser Leu Ile Asp Leu Gly His Arg Phe Arg
   385                 390                 395                 400
   Leu Ile Val Asn Glu Val Asp Ala Val Lys Pro Glu His Asp Met Pro
                   405                 410                 415
   Lys Leu Pro Val Ala Arg Ile Leu Trp Lys Pro Arg Pro Ser Leu Arg
               420                 425                 430
   Asp Ser Ala Glu Ala Trp Ile Leu Ala Gly Gly Ala His His Thr Cys
           435                 440                 445
   Phe Ser Phe Ala Val Thr Thr Glu Gln Leu Gln Asp Phe Ala Glu Met
       450                 455                 460
   Ala Gly Ile Glu Cys Val Val Ile Asn Glu His Thr Ser Val Ser Ser
   465                 470                 475                 480
   Phe Lys Asn Glu Leu Lys Trp Asn Glu Val Phe Trp Arg Gly Arg
                   485                 490                 495
<210> 5
   <211> 1491
   <212> DNA
   <213> Artificial
<220>
   <223> mutated L-arabinose isomerase from G. stearothermophilus
<400> 5
   atgctgtcat tacgtcctta tgaattttgg tttgtaacgg gaagccagca cttgtacgga 60
   gaagaagcat taaagcaagt tgaagagcat tcaatgatga ttgtcaatga gctgaatcaa 120
   gattcagtgt tcccgttccc acttgttttc aaatcagttg tcacaacgcc agaggaaatc 180
   cggcgcgttt gccttgaggc gaatgcgagc gaacaatgcg ctggggtcat cacttggatg 240
   catacattct cgccagcgaa gatgtggatt ggcggccttt tggagctgcg aaaaccgtta 300
   ttgcatcttc acactcaatt taaccgtgat attccgtggg acagcatcga tatggacttt 360
   atgaacttaa accaatcggc tcacggtgac cgggaatacg gatttatcgg cgcgagaatg 420
   ggcgtggccc ggaaagtggt ggtcgggcac tgggaagacc cagaagtccg cgagcggctg 480
   gcgaaatgga tgcggacggc tgtcgcgttt gcggaaagcc gcaacctaaa agtggctcgt 540
   tttggcgaca acatgcgtga agtggctgtg acggaagggg acaaagtcgg agcgcaaatt 600
   caattcggct ggtcggtcaa cggctatggc atcggggatt tggtgcaata catccgcgat 660
   gtttctgaac aaaaagtgaa cgagttgctc gatgaatacg aggagctgta cgacattgta 720
   cccgccggcc gccaagaagg gcccgttcgc gaatcaattc gtgaacaggc gcggattgaa 780
   ctcgggctga aagccttttt gcaggatggg aactttaccg ctttcacgac gacgttcgag 840
   gacttgcatg ggatgaagca gctgccggga cttgccgttc agcgactcat ggcggaagga 900
   tacggttttg gaggcgaggg agactggaaa acggctgcgc ttgtccggct aatgaaagtg 960
   atggctgatg gaaaagggac gtcatttatg gaagattaca cgtaccacct tgagccgggt 1020
   aatgaactga ttcttggcgc ccacatgttg gaagtagatc cgactattgc agcgacccgg 1080
   ccgcgaatcg aagtccatcc gctttccatc ggtggaaaag aagatccagt gcgtctcgtg 1140
   tttgacggcg gcgagggtgc ggcggtcaac gcgtcattga tcgacttagg gcaccgtttc 1200
   cgactcatcg tcaatgaagt cgatgcggtg aaaccggaac acgacctgcc gaaattacca 1260
   gtcgcccgca ttttatggaa gcctcgcccg tcgttccgcg actccgctga agcatggatt 1320
   ttagctggcg gcgcccacca tacggtgttc tcatttgcgg ttacaacaga acagctgcaa 1380
   gactttgcgg aaattgcagg gattgaactg gtcgtgatca atgaacatac gtccgtctcg 1440
   tccttcaaaa atgaactgaa atggaacgaa gtgttttggc gggggcggta a 1491
<210> 6
   <211> 1488
   <212> DNA
   <213> Artificial
<220>
   <223> mutated L-arabinose isomerase from G. stearothermophilus
<400> 6
   atgtcattac gtccttatga attttggttt gtaacgggaa gccagcactt gtacggagaa      60
   gaagcattaa agcaagttga agagcattca agcaaaattg tcaatgagct gaatcaagat     120
   tcagtgttcc cgttcccact tgttttcaaa tcagttgtca caacgccaga ggaaatccgg     180
   cgcgttttcc ttgaggcgaa tgcgagcgaa caatgcgctg gggtcatcac ttggctgcat     240
   acattctcgc cagcgaagag ctggattggc ggccttttgg agctgcgaaa accgttattg     300
   catcttcaca ctcaatttaa ccgtgatatt ccgtgggaca gcatcgatat ggactttatg     360
   aacttaaacc aatcggctca cggtgaccgg gaatacggat ttatcggcgc gagactgggc     420
   gtggcccgga aagtggtggt cgggcactgg gaagacccag aagtccgcga gcggctggcg     480
   aaatggatgc ggacggctgt cgcgtttgcg gaaagccgca acctaaaagt ggctcgtttt     540
   ggcgacaaca tgcgtgaagt ggctgtgacg gaaggggaca aagtcggagc gcaaattcaa     600
   ttcggctggt cggtcaacac ctatggcatc ggggatttgg tgcaatacat ccgcgatgtt     660
   tctgaacaaa aagtgaacga gttgctcgat gaatacgagg agctgtacga cattgtaccc     720
   gccggccgcc aagaagggcc cgttcgcgaa tcaattcgtg aacaggcgcg gattgaactc     780
   gggctgaaag cctttttgca ggatgggaac tttaccgctt tcacgacgac gttcgaggac     840
   ttgcatgggc tgaagcagct cccgggactt gccgttcagc gactcctggc ggaaggatac     900
   ggttttggag gcgagggaga ctggaaaacg gctgcgcttg tccggctact gaaagtgctg     960
   gctgatggaa aagggacgtc atttatggaa gattacacgt accaccttga gccgggtaat    1020
   gaaatgattc ttggcgccca catgttggaa gtatgcccga ctattgcagc gacccggccg    1080
   cgaatcgaag tccatccgct ttccatcggt ggaaaagaag atccagcccg tctcgtgttt    1140
   gacggcggcg agggtgcggc ggtcaacgcg tcattgatcg acttagggca ccgtttccga    1200
   ctcatcgtca atgaagtcga tgcggtgaaa ccggaacacg acatgccgaa attaccagtc    1260
   gcccgcattt tatggaagcc tcgcccgtcg ctccgcgact ccgctgaagc atggatttta    1320
   gctggcggcg cccaccatac gtgcttctca tttgcggtta caacagaaca gctgcaagac    1380
   tttgcggaaa tggcagggat tgaatgtgtc gtgatcaatg aacatacgtc cgtctcgtcc    1440
   ttcaaaaatg aactgaaatg gaacgaagtg ttttggcggg ggcggtaa                 1488
<210> 7
   <211> 1488
   <212> DNA
   <213> Artificial
<220>
   <223> mutated L-arabinose isomerase from G. stearothermophilus
<400> 7
   atgtcattac gtccttatga attttggttt gtaacgggaa gccagcactt gtacggagaa      60
   gaagcattaa agcaagttga agagcattca agcaaaattg tcaatgagct gaatcaagat     120
   tcagtgttcc cgttcccact tgttttcaaa tcagttgtca caacgccaga ggaaatccgg     180
   cgcgttttcc ttgaggcgaa tgcgagcgaa caatgcgctg gggtcatcac ttggctgcat      240
   acattctcgc cagcgaagag ctggattggc ggccttttgg agctgcgaaa accgttattg      300
   catcttcaca ctcaatttaa ccgtgatatt ccgtgggaca gcatcgatat ggactttatg     360
   aacttaaacc aatcggctca cggtgaccgg gaatacggat ttatcggcgc gagactgggc     420
   gtggcccgga aagtggtggt cgggcactgg gaagacccag aagtccgcga gcggctggcg     480
   aaatggatgc ggacggctgt cgcgtttgcg gaaagccgca acctaaaagt ggctcgtttt     540
   ggcgacaaca tgcgtgaagt ggctgtgacg gaaggggaca aagtcggagc gcaaattcaa     600
   ttcggctggt cggtcaacac ctatggcatc ggggatttgg tgcaatacat ccgcgatgtt     660
   tctgaacaaa aagtgaacga gttgctcgat gaatacgagg agctgtacga cattgtaccc     720
   gccggccgcc aagaagggcc cgttcgcgaa tcaattcgtg aacaggcgcg gattgaactc     780
   gggctgaaag cctttttgca ggatgggaac tttaccgctt tcacgacgac gttcgaggac     840
   ttgcatggga tgaagcagct gccgggactt gccgttcagc gactcatggc ggaaggatac     900
   ggttttggag gcgagggaga ctggaaaacg gctgcgcttg tccggctaat gaaagtgatg     960
   gctgatggaa aagggacgtc atttatggaa gattacacgt accaccttga gccgggtaat    1020
   gaaatgattc ttggcgccca catgttggaa gtatgcccga ctattgcagc gacccggccg    1080
   cgaatcgaag tccatccgct ttccatcggt ggaaaagaag atccagcccg tctcgtgttt    1140
   gacggcggcg agggtgcggc ggtcaacgcg tcattgatcg acttagggca ccgtttccga    1200
   ctcatcgtca atgaagtcga tgcggtgaaa ccggaacacg acatgccgaa attaccagtc    1260
   gcccgcattt tatggaagcc tcgcccgtcg ctccgcgact ccgctgaagc atggatttta    1320
   gctggcggcg cccaccatac gtgcttctca tttgcggtta caacagaaca gctgcaagac    1380
   tttgcggaaa tggcagggat tgaatgtgtc gtgatcaatg aacatacgtc cgtctcgtcc    1440
   ttcaaaaatg aactgaaatg gaacgaagtg ttttggcggg ggcggtaa                 1488
<210> 8
   <211> 495
   <212> PRT
   <213> Artificial
<220>
   <223> mutated L-arabinose isomerase from G. stearothermophilus
<400> 8
   Met Ser Leu Arg Pro Tyr Glu Phe Trp Phe Val Thr Gly Ser Gln His
   1               5                   10                  15
   Leu Tyr Gly Glu Glu Ala Leu Lys Gln Val Glu Glu His Ser Ser Lys
               20                  25                  30
   Ile Val Asn Glu Leu Asn Gln Asp Ser Val Phe Pro Phe Pro Leu Val
           35                  40                  45
   Phe Lys Ser Val Val Thr Thr Pro Glu Glu Ile Arg Arg Val Phe Leu
       50                  55                  60
   Glu Ala Asn Ala Ser Glu Gln Cys Ala Gly Val Ile Thr Trp Leu His
   65                  70                  75                  80
   Thr Phe Ser Pro Ala Lys Ser Trp Ile Gly Gly Leu Leu Glu Leu Arg
                   85                  90                  95
   Lys Pro Leu Leu His Leu His Thr Gln Phe Asn Arg Asp Ile Pro Trp
               100                 105                 110
   Asp Ser Ile Asp Met Asp Phe Met Asn Leu Asn Gln Ser Ala His Gly
           115                 120                 125
   Asp Arg Glu Tyr Gly Phe Ile Gly Ala Arg Leu Gly Val Ala Arg Lys
       130                 135                 140
   Val Val Val Gly His Trp Glu Asp Pro Glu Val Arg Glu Arg Leu Ala
   145                 150                 155                 160
   Lys Trp Met Arg Thr Ala Val Ala Phe Ala Glu Ser Arg Asn Leu Lys
                   165                 170                 175
   Val Ala Arg Phe Gly Asp Asn Met Arg Glu Val Ala Val Thr Glu Gly
               180                 185                 190
   Asp Lys Val Gly Ala Gln Ile Gln Phe Gly Trp Ser Val Asn Thr Tyr
           195                 200                 205
   Gly Ile Gly Asp Leu Val Gln Tyr Ile Arg Asp Val Ser Glu Gln Lys
       210                 215                 220
   Val Asn Glu Leu Leu Asp Glu Tyr Glu Glu Leu Tyr Asp Ile Val Pro
   225                 230                 235                 240
   Ala Gly Arg Gln Glu Gly Pro Val Arg Glu Ser Ile Arg Glu Gln Ala
                   245                 250                 255
   Arg Ile Glu Leu Gly Leu Lys Ala Phe Leu Gln Asp Gly Asn Phe Thr
               260                 265                 270
   Ala Phe Thr Thr Thr Phe Glu Asp Leu His Gly Leu Lys Gln Leu Pro
           275                 280                 285
   Gly Leu Ala Val Gln Arg Leu Leu Ala Glu Gly Tyr Gly Phe Gly Gly
       290                 295                 300
   Glu Gly Asp Trp Lys Thr Ala Ala Leu Val Arg Leu Leu Lys Val Leu
   305                 310                 315                 320
   Ala Asp Gly Lys Gly Thr Ser Phe Met Glu Asp Tyr Thr Tyr His Leu
                   325                 330                 335
   Glu Pro Gly Asn Glu Leu Ile Leu Gly Ala His Met Leu Glu Val Asp
               340                 345                 350
   Pro Thr Ile Ala Ala Thr Arg Pro Arg Ile Glu Val His Pro Leu Ser
           355                 360                 365
   Ile Gly Gly Lys Glu Asp Pro Val Arg Leu Val Phe Asp Gly Gly Glu
       370                 375                 380
   Gly Ala Ala Val Asn Ala Ser Leu Ile Asp Leu Gly His Arg Phe Arg
   385                 390                 395                 400
   Leu Ile Val Asn Glu Val Asp Ala Val Lys Pro Glu His Asp Leu Pro
                   405                 410                 415
   Lys Leu Pro Val Ala Arg Ile Leu Trp Lys Pro Arg Pro Ser Phe Arg
               420                 425                 430
   Asp Ser Ala Glu Ala Trp Ile Leu Ala Gly Gly Ala His His Thr Val
           435                 440                 445
   Phe Ser Phe Ala Val Thr Thr Glu Gln Leu Gln Asp Phe Ala Glu Ile
       450                 455                 460
   Ala Gly Ile Glu Leu Val Val Ile Asn Glu His Thr Ser Val Ser Ser
   465                 470                 475                 480
   Phe Lys Asn Glu Leu Lys Trp Asn Glu Val Phe Trp Arg Gly Arg
                   485                 490                 495

## Description

### Field of the invention

The present invention relates to the fields of industrial enzymes, protein engineering and the production sweeteners. In particular, the invention relates to the production of D-tagatose from D-galactose using an arabinose isomerase for isomerisation of D-galactose to D-tagatose.

### Background of the invention

Tagatose, a keto-sugar of galactose, serves as low calorigenic sweetener, because when ingested in the body only approximately 20% of the ingested tagatose is absorbed in the small intestine. The absorbed part is metabolized in the liver by the same pathway as fructose. Additionally, in contrast to sugar alcohols, the most prevalent sugar-substitutes in current use, and which have a laxative effect causing diarrhea at a certain dose, tagatose enjoys the advantage of not having such a laxative effect. Another advantage of tagatose is that, in contrast to sugar alcohols, tagatose can give appropriate flavours upon food processing because of its brown change upon heating like sugar. These properties have attracted great attention to tagatose as a safe low-caloric sugar substitute with a great market potential (Zehener, 1988, EP 257626; Marzur, 1989, EP 0341062A2). In addition, it has been reported that tagatose can be employed as an intermediate for the preparation of useful optically active isomers, detergents and cosmetics. Also, it has been reported that tagatose can be used as an additive or raw material for the synthesis of drugs. For example, its ability to lower blood sugar level renders tagatose a therapeutic and preventive agent for diabetes, and a low caloric diet agent.

D-tagatose is generally produced by chemical or biological methods. One of the methods for manufacturing tagatose is an enzymatic method in which galactose is converted into tagatose via conversion of aldose or aldose derivatives into ketose or ketose derivatives. Especially, it has been reported that L-arabinose isomerase which catalyzes the conversion reaction of L-arabinose into L-ribulose can be employed for production of tagatose *in vitro* using galactose as a substrate. However, the yield of tagatose produced by arabinose isomerase from galactose is as low as 20%, hindering industrial application of conversion process of galactose into tagatose.

L-arabinose isomerase (AI, EC 5.3.1.4) is an enzyme that exhibits different catalytic actions *in vivo* and *in vitro*. In nature AI mediates the conversion from L-arabinose to L-ribulose. However, *in vitro* AI can also be applied to catalyse the conversion from D-galactose to D-tagatose, whereby the arabinose isomerase acts as a D-galactose isomerase. The equilibrium of the isomerisation between galactose and tagatose has been reported to vary with reaction temperature. Typically, the reaction shifts towards the ketose, *i.e.,* tagatose, as the reaction temperature increases. Several AI from both mesophilic and (hyper)thermophilic species have been characterized and subjected to studies to increase the D-tagatose production.

WO 03/008593 discloses an isolated L-arabinose isomerase active enzyme derived from a *Thermoanaerobacter* species wherein the nucleic acid coding for L-arabinose isomerase is a wild type nucleic acid isolated from a *Thermoanaerobacter* species.

Lee et al. (2005) have studied the AI of *Geobacillus stearothermophilus* (GSAI). The optimal temperature for GSAI is 60°C, which indicates that GSAI is thermophilic. The enzyme also remains active at higher temperatures. No loss of activity was seen after incubation for 2h at 70°C; and after 52 min at 80°C activity was only halved. The optimal pH is reported to be 8.0. L-arabitol and ribitol are inhibitors of the isomerisation reaction (Kim et al. 2006). Lee and co-workers (2005) demonstrated that GSAI is a homotetramer.

Kim et al. (2001) disclosed a mutated AI derived from a thermostable AI from hot-spring bacteria with 11-fold higher reaction rate than the original. Kim and co-workers (2006) disclosed another mutant AI derived from *G. stearothermophilus* that was found to result in an increased rate of D-tagatose production. This mutant has an optimal activity at 65°C, pH of 8.0 and 1.0 mM Co²⁺, which activity is 1.9 times higher than that of the wild type enzyme (Kim et al. 2006).

In US patent application No. 20030175909, thermostable galactose isomerases and production of tagatose using the galactose isomerases are disclosed. In US patent application No. 20100173366 the identification of a gene encoding an AI of *Bacillus stearothermophilus* strain US 100 is disclosed. Similarly, in US patent 6,933,138 and US 7,501,246 a thermostable AI from *Thermotoga neapolitana* 5068 and a process for preparing D-tagatose is disclosed. In US 7,052,898 a thermostable isomerase from a thermophilic source for producing tagatose is disclosed. Other patent applications that disclose AI are for example US 20080124770 and US 2008124771.

However, there is still a need in the art for enzymes and processes which can produce D-tagatose with high yield under tagatose production conditions at 60 degrees Celsius.

### Description of the invention

### Summary of the invention

In a first aspect, the present invention relates to a protein comprising an amino acid sequence of SEQ ID NO: 3 or SEQ ID NO: 4 or comprising an amino acid sequence with at least 95% amino acid identity with SEQ ID NO: 3 or SEQ ID NO: 4. Preferably, the protein has D-galactose isomerase activity.

In a preferred embodiment, a protein according to the invention comprises an amino acid sequence which has at least 95% amino acid identity with SEQ ID NO: 1, wherein the amino acid sequence has a) conservative amino acid substitutions in the positions 32, 33, 64, 80, 88, 140 and 208, and, b) a conservative amino acid substitution or deletion of the amino acid at position 2, and wherein the amino acid substitutions are M32S, M33K, C64F, M80L, M88S, M140L, G208T and the deletion is deletion of Leu2.

In a second aspect, the present invention relates to a nucleic acid molecule encoding for the protein of the present invention.

In a third aspect, the present invention relates to a nucleic acid construct, comprising a nucleic acid molecule according to the invention operably linked to a promoter for expression in a host cell.

In a fourth aspect, the present invention relates to a host cell comprising a nucleic acid construct according to the invention. In a preferred embodiment, the host cell is *Escherichia coli*, more preferably *E. coli* strain BL21.

In a fifth aspect, the present invention relates to a method for the production of a protein according to the invention, the method comprising the steps of: a) culturing a host cell of the present invention under conditions conducive of producing the protein; and b) optionally isolating the protein.

In a sixth aspect, the present invention relates to a method for the production of alginate beads comprising a protein according to the invention, wherein the method comprises the steps of: a) culturing a host cell of claim 5 or 6 under conditions conducive of producing the protein according to claim 1 or claim 2 to obtain a cell culture comprising the host cell comprising a protein; b) adding 0.5 - 10 % (w/v) alginate to the cell culture obtained in step a) and incubate for 1 - 24 hours at 45 - 100°C ; c) adding the mixture obtained in step b) dropwise into a solution comprising divalent cations, to obtain beads with a mean diameter of 2 to 3 mm; d) incubating the solution comprising the beads obtained in step c) at 1 - 10 °C for 1 - 36 hours harden the beads; and e) isolating the hardened beads obtained in step d), preferably by filtration and/or centrifugation.

In a seventh aspect, the present invention relates to a solid support onto or within which a protein according to the invention is immobilized. In a preferred embodiment, the solid support is selected from the group consisting of: alginate, chitin, chitosan, cellusose, and silica. In a more preferred embodiment, the solid support is an alginate bead within which a protein according to the invention is immobilized.

In a further aspect, the present invention relates to an alginate bead comprising a protein according to invention, preferably obtainable by a method for the production of alginate beads according to the invention.

In another aspect, the present invention relates to a method for the production of D-tagatose, the method comprising the steps of: i) incubating a sample comprising galactose with a protein according to the invention, a solid support according to the invention or an alginate bead according to the invention, under conditions conducive of production of D-tagatose; and ii) optionally at least one of purification and recovery of the tagatose. In a preferred embodiment, the method further comprises the steps of purifying the D-tagatose using one or more methods selected from the group consisting of passing over an active carbon column, ion exchange chromatography, SMB, crystalisation and drying.

In yet another aspect, the present invention relates to use of a protein according to the invention, a solid support according to the invention or an alginate bead according to the invention, in the enzymatic conversion of D-galactose into D-tagatose.

### Definitions

The terms "tagatose" or "D-tagatose" are used interchangeably herein and are understood to indicate a rare natural hexoketose, which is an isomer of d-galactose and has the formula:

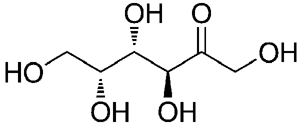

D-tagatose occurs naturally in Sterculia setigera gum, and it is also found in small quantities in various foods such as sterilized and powdered cow's milk, hot cocoa, and a variety of cheeses, yogurts, and other dairy products. Other synonyms that are known are: lyxo-Hexulose, D-lyxo-2-Hexulose, D-tag, D-lyxo-hex-2-ulose and lyxo-2-Hexulose.

Similarly, the terms "galactose" and "D-galactose" are used interchangeably herein. D-galactose (aldohexose galacto-hexose) is a monosaccharide that can be converted to UDP-glucose via the Leloir pathway. Galactose is found in dairy products, sugar beets, and other gums and mucilages. D-galactose is widely distributed in combined form in plants, animals and microorganisms as a constituent of oligo- and polysaccharides. It is also synthesized by the body, where it forms part of glycolipids and glycoproteins in several tissues.

The terms peptide and polypeptide are used synonymously herein to refer to this class of compounds without restriction as to size. The largest members of this class are referred to as proteins. In the context of the invention, a peptide (or a protein) is represented by an amino acid sequence.

In the context of the invention, a nucleic acid molecule is represented by a nucleic acid or nucleotide sequence which encodes a protein or a polypeptide or a protein fragment. A nucleic acid molecule may comprise a regulatory region.

It is to be understood that each nucleic acid molecule or peptide (or protein) as identified herein by a given Sequence Identity Number (SEQ ID NO) is not limited to this specific sequence as disclosed, unless otherwise indicated. Each gene sequence or nucleotide sequence as identified herein encodes a given peptide or is itself a peptide.

Each nucleotide sequence or amino acid sequence described herein by virtue of its identity or similarity percentage with a given nucleotide sequence or amino acid sequence respectively has in a further preferred embodiment an identity or a similarity of at least 85%, 90%, 95%, 96%, 97%, 98%, 99% or 100% identity or similarity with the given nucleotide or amino acid sequence respectively. In a preferred embodiment, sequence identity or similarity is determined by comparing the whole length of the sequences as identified herein.

"Sequence identity" is herein defined as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO or on part thereof. Part thereof preferably means at least 50%, 60%, 70%, 80%, 90%, or 100% of both SEQ ID NO. In the art, "identity" also means the degree of sequence relatedness between amino acid or nucleic acid sequences, as the case may be, as determined by the match between strings of such sequences.

"Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

The terms "sequence identity" or "sequence similarity" means that two (poly)peptide or two nucleotide sequences, when optimally aligned, preferably over the entire length (of at least the shortest sequence in the comparison) and maximizing the number of matches and minimizes the number of gaps such as by the programs ClustalW (1.83), GAP or BESTFIT using default parameters, share at least a certain percentage of sequence identity or similarity as defined elsewhere herein. Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

GAP uses the Needleman and Wunsch global alignment algorithm to align two sequences over their entire length, maximizing the number of matches and minimizes the number of gaps. Generally, the GAP default parameters are used, with a gap creation penalty = 50 is ClustalW (1.83) using a Blosum matrix and default settings (Gap opening penalty: 10; Gap extension penalty: 0.05).

Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). A preferred multiple alignment program for aligning protein sequences of the invention EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blossum62 for proteins and DNAFull for DNA). When sequences have a substantially different overall lengths, local alignments, such as those using the Smith Waterman algorithm, are preferred. Alternatively percentage similarity or identity may be determined by searching against public databases, using algorithms such as FASTA, BLAST, etc.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains and that can be placed in groups of interchangeable amino acids. For example, a group of amino acids having aliphatic and/or non-polar side chains is glycine, alanine, valine, leucine, isoleucine and methionine; a group of amino acids having a polar side chain is asparagine, cysteine, glutamine, serine, threonine and tyrosine; a group of amino acids having aliphatic-hydroxyl side chains is serine and threonine; a group of amino acids having small side chains is alanine, serine, threonine, methionine and glycine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; a group of amino acids having acidic side chains is aspartic acid and glutamic acid; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg, gln or glu; Met to leu or ile; Phe to met, leu or tyr; Ser to thr or gly; Thr to ser or val; Trp to tyr; Tyr to trp or phe; and, Val to ile or leu.

A nucleic acid construct comprises a nucleotide sequence encoding a peptide or protein as defined herein. A nucleic acid construct comprising a nucleic acid molecule coding for a given peptide or protein as defined herein will ensure expression of the given nucleic acid molecule, and of the corresponding peptide or protein in a host cell. In an embodiment, a nucleic acid construct comprises more than one nucleic acid molecule, each nucleic acid molecule coding for a given peptide or protein. In an even more preferred embodiment, a nucleic acid construct comprises two, three, four nucleic acid molecules, each nucleic acid molecule coding for a given peptide or protein. In a preferred embodiment, a nucleic acid construct comprises an expression cassette, said expression cassette comprising each needed nucleic acid molecule. Each nucleic acid molecule is operably linked with other nucleic acid molecule present. Most preferably, a suitable promoter is operably linked with the expression cassette to ensure expression of the nucleic acid molecule in a subject.

### Detailed description of the invention

L-Arabinose isomerase (AI, EC 5.3.1.4) is an enzyme that can exhibit different catalytic actions *in vivo* and *in vitro*. In nature AI mediates the conversion from L-arabinose to L-ribulose. However, *in vitro* AI can also be applied to catalyse the conversion from D-galactose to D-tagatose, whereby the enzyme thus acts as a D-galactose isomerase.

The present invention relates to D-galactose isomerases, thus a protein of the present invention has D-galactose isomerase activity. Disclosed are mutant D-galactose isomerases (GI) from *Geobacillus stearothermophilus*. Specific amino acid residues in the sequence of GI have been selectively replaced to limit the number of potential sites of oxidation, which the inventors consider to be a main parameter contributing to enzyme loss of activity at 60°C. Some replacements appear to improve the activity of the enzyme once encapsulated and used on a continuous flow column system for galactose conversion into tagatose. Some replacements did not result in improved D-galactose isomerase activity. Some of the resulting enzymes show increased activity under tagatose production conditions at 60°C. Three of these enzymes are presented in the present application (see Examples).

In general, the tertiary structure of AI is composed of three domains: an N-terminal domain, a central part and a C-terminal domain. The N-terminal domain is similar to a Rossmann fold: a central beta-sheet composed of 5 parallel beta-strands is sandwiched between 2 alpha-helices on one side and 3 alpha-helices on its other side. The central domain, also resembles a Rossmann fold, but the beta-sheet is composed of only 4 parallel beta-strands. The C-terminal domain, forms a beta-barrel like structure which is composed of 6 anti-parallel beta-strands. The C-terminal and N-terminal ends of the barrel are formed by helices and long loops, respectively. The active site is located between the central and the C-terminal domains with most of the contributing residues originating from the C-terminal domain (Manjasetty and Chance 2006; Takeda et al. 2010).

The quaternary structure of AI is generally dependent on the thermostability of the AI. The biological unit of AI from mesophilic species is a hexamer while AI of (hyper)thermophilic species is shown to be a tetramer (Manjasetty and Chance 2006). The hexamer is formed by a dimer of trimers: 2 trimers are stacked against each other. The trimer has a triangular prismatic shape. When looking at the quaternary structure, the active site is located at the interface between 2 monomers within the trimer. The subunit interface of the trimer contains several salt bridges, H bonds and hydrophobic interactions. Most of the residues involved are conserved; both within the mesophilic and thermophilic enzymes. The trimer-trimer interface is mainly composed of hydrophobic interactions (Manjasetty and Chance 2006). Unfortunately, little is known on the composition of the tetramer.

The object of the present invention was to increase at least one of the stability and activity of the D-galactose isomerase of *Geobacillus stearothermophilus* (GSGI; SEQ ID NO:1) and preferably such improved GSGIs can be employed in processes that result in a higher yield of D-tagatose. AI of *G. stearothermophilus* was used because the AI is well described in scientific literature and because it is from a thermophilic microorganism.

### Protein

In a first aspect, the present invention relates to a protein comprising an amino acid sequence of any one of SEQ ID NO: 3 or SEQ ID NO: 4 or comprising an amino acid sequence with at least 95%, 96%, 97%, 98%, or 99% amino acid identity to an amino acid sequence of any one of SEQ ID NO: 3 or SEQ ID NO: 4. Preferably, the protein has tetramer formation. Preferably, the protein has D-galactose isomerase activity or, alternatively said, is a D-galactose isomerase.

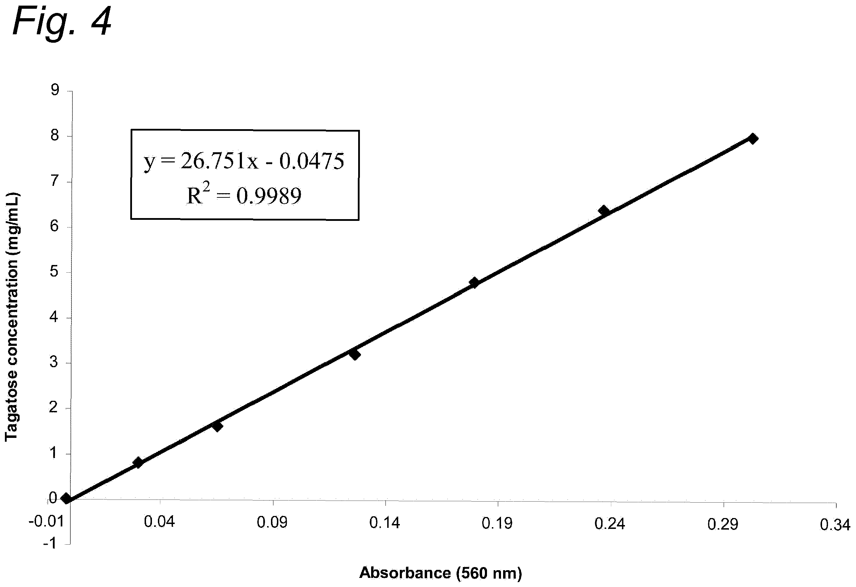

To determine whether a protein has D-galactose isomerase activity, D-galactose isomerase activity in fermentation is preferably measured using the protocol based on the method of Dische and Borenfreund (J. Biol. Chem. (1951) 192:583-587). The D-galactose isomerase activity can be assayed based on the appearance of keto-hexose (D-tagatose) after incubation in the presence of the substrate aldo-hexose (D-galactose). The conversion of galactose into tagatose is highly specific, no other sugars are produced. D-tagatose can be measured colorimetrically utilizing cysteine-carbazole-sulfuric acid as a reagent. The amount of tagatose (ketose) is a measure for the D-galactose isomerase activity and is determined with a spectrophotometer at a wavelength of 560 nm after a colour forming of ketoses with a cysteine / carbazole / sulfuric acid reagent. Samples are preferably analysed in duplicate, more preferably in triplicate, utilizing a blank to correct for substances which may interfere with the colorimetric ketose determination. This method, is a relative method and the results are related to a tagatose standard (Figure 4). The method is preferably performed in a 96-wells microtiterplate in a final volume of 135 µl as follows: first, 100 µl of a sample comprising approximately 25 µM of the protein to be tested for AI activity is added to 900 µl of test solution (385 mg/ml galactose; 1 mM MnCl₂; 50 mM Tris [pH 7.5]) in capped sample tubes, mixed and allowed to incubate for 2 hours at 60°C. Subsequently, each sample is diluted 100 times in water and 15 µl of the dilution is transferred to a 96-well microtitre plate. In addition, a standard curve is prepared from tagatose solutions of 4, 8, 16, 32, 40 and 160 g/l, which are diluted 100 times and are also transferred to the microtitre plate. To each of the wells, 10 µl of L-cystein (47 mM) is added. The microtitre plate is shaken for 2 minutes at 800 rpm and subsequently 100 µl of sulfuric acid (14 M) is added and the plate is shaken for 5 minutes at 800 rpm. Finally, 10 µl of carbazol (3.6 mM) is transferred to each well and the plate is shaken for 5 minutes at 800 rpm. The plate is then allowed to incubate for 30 min at 25°C. Immediately after incubation, the colour intensity in the wells is determined using spectrophotometry at a wavelength of 560 nm. Using the standard curve, the tagatose concentration and the D-galactose isomerase activity can be determined (figure 4). One unit of D-galactose isomerase is herein defined as the amount of protein that is required to produce 1 gram of D-tagatose per liter from D-galactose following incubation for 2 hours at 60°C under assay conditions.

For determination of the D-galactose isomerase activity of AI encapsulated in alginate beads, the enzymatic step is skipped and instead the samples are diluted 500 times in water and transferred directly to the microtitre plate.

A protein according to the invention, is defined to have D-galactose isomerase activity if, when the protein is produced in *E. coli* BL21 in a 15 L fermenter as described in Example 2, the specific activity (U/g biomass) measured in the pellet is larger than the specific activity measured in a pellet of negative control sample (i.e. a pellet of *E. coli* BL21 containing the 'empty' vector). A protein according to the invention, when produced in *E. coli* BL21 in a 15 L fermenter as described in Example 2, preferably has a specific activity (U/g biomass) in the pellet that is at least 105, 110, 115, 120, 125, 130, 135, 140, 145, 150, 155, 160, 165% of the specific activity of the pellet of the wild type enzyme (SEQ ID NO:1). The specific activity can be less than 250, 220, 200, 190, 185, 180, 175% of the specific activity of the pellet of the wild type enzyme (SEQ ID NO:1). In a preferred embodiment, the specific activity is between 105 and 250%, preferably between 110 and 200%, more preferably between 120 - 200%, 120 - 180%, 130 - 180, 140 - 180%, 150 - 180%, 160 - 180% of the specific activity of the wild type enzyme (SEQ ID NO:1).

The above-described method for tagatose detection can be applied for the determination of relative D-galactose isomerase activity in fermentation, such as for example in the pellet as demonstrated in Example 3, in downstream processing (DSP) and in immobilized samples.

In a preferred embodiment, a protein according to the invention consists of an amino acid sequence of any one of SEQ ID NO: 3 or SEQ ID NO: 4 or consists of an amino acid sequence with at least 95%, 96%, 97%, 98%, or 99% amino acid identity or similarity with any one of SEQ ID NO: 3 or SEQ ID NO: 4. Preferably, the protein has D-galactose isomerase activity or, alternatively said, is an D-galactose isomerase. A preferred method to determine whether a protein has D-galactose isomerase activity has been described above. In a preferred embodiment, a protein according to the invention comprises or consists of an amino acid sequence which has with at least 95%, 96%, 97%, 98%, or 99% amino acid identity with SEQ ID NO: 1 and wherein the amino acid sequence has a) one or more amino acid substitutions in the following positions: 32, 33, 64, 80, 88, 140, 208, 285, 297, 318, 321, 343, 353, 377, 416, 432, 449, 465 and 470 and, b) an amino acid substitution or deletion of the amino acid at position 2.

Preferably, the amino acid substitutions are conservative amino acid substitutions as herein defined above. Preferred positions for conservative amino acid substitutions are the following residues: methionine residue on any one of the positions 32, 80, 88, 140, 285, 297, 318, 321, 343, 416 and/or the glycine residue on position 208 and/or the alanine residue on position 380 and combinations thereof. Preferred substitutions/deletions are selected from the group consisting of: deletion of L2 (L2-), M32S, M80L, M88S, M140L, M285L, M297L, M318L, M321L, M343L, M416L, G208T, A370V and combinations thereof. Conservative substitutions at positions 32, 80, 88, 140, 285, 297, 318, 321, 343, 416 are likely to remove a possible site for oxidation. Conservative substitution at position 208, preferably to Thr removes the flexibility that is allowed by glycine. Conservative substitution at position 377, preferably to valine, is likely to result in the core of the AI to be more compact. Other preferred mutations are removal of the methionine at position 1, since it seems not to be involved in any secondary structure; mutation of methionine at position 33, preferably to lysine, since this is likely to remove a site for oxidation, mutation of cysteine at position 64, preferably to phenylalanine, in order to allow several hydrophobic interactions, mutation of cysteine, preferably to asparagine to remove a possible site of oxidation and to allow a salt bridge with the lysine residue at position 319, mutation of the leucine residue at position 432, preferably to phenylalanine which is believed to allow a tighter packing of the AI.

In a preferred embodiment, a protein according to the invention comprises or consists of an amino acid sequence which has with at least 95%, 96%, 97%, 98%, or 99% amino acid identity with SEQ ID NO: 1, and wherein the amino acid sequence has at least one of a) amino acid substitutions in one or more or all of the positions 32, 33, 64, 80, 88, 140 and 208, and b) a deletion of the amino acid at position 2. Preferably the protein additionally has amino acid substitutions in one or more or all of in the positions 285, 297, 318 and 321. Preferably these amino acid substitutions are conservative substitutions as herein defined above. More preferably the amino acid substitutions are one or more or all of M32S, M33K, C64F, M80L, M88S, M140L, G208T, M285L, M297L, M318L and M321L and the deletion is preferably deletion of Leu2 (L2-). Alternatively, or additionally the protein has amino acid substitutions in one or more or all of in the positions 343, 353, 377, 416, 432, 4492, 465 and 470. Preferably these amino acid substitutions are conservative substitutions as herein defined above. More preferably the amino acid substitutions are one or more or all of M343L, C353D, A377V, M416L, L432F, C449V, M465I and C470L.

In a preferred embodiment, amino acid residues Glu306, Glu331, Tyr333, His348, His447 of SEQ ID NO: 1 should not be substituted by alternative or conservative amino acid residues, since the residues at positions 306, 331, 348 and 447 are likely to be catalytic residues and the aromatic residue at position 333 is likely to be necessary to precisely position the sugar in the catalytic site of the enzyme. It is further preferred that, other than substitutions in positions in SEQ ID NO: 1 that are specifically indicated herein above, invariable amino acid residues in the sequence alignment of Figure 4 of Rhimi et al. (2007, J. Bacteriol. 189(9):3556-3563) are not altered in the proteins of the invention.

In bacteria, the initiation codon of the nucleic acid sequence encoding an amino acid sequence, codes for a formylmethionine. This formylmethionine is removed from the majority of proteins (both endogenous host proteins and recombinant proteins) by methionine aminopeptidase (Ben-Bassat et al. (1987) Journal of Bacteriology 169 (2):751-757). The amino acid sequences presented in the sequence listing attached to the present application start with this formylmethionine. It is however understood that a protein having D-galactose isomerase activity of the invention includes a protein lacking the amino terminal formylmethionine. Likewise, in a composition comprising a protein having D-galactose isomerase activity of the invention, at least a fraction of the protein in the composition can be a protein lacking the amino terminal formylmethionine.

The *in vivo* stability of proteins can be dependent on the type of amino acid that is present in the NH₂-terminal position (Tobias et al. (1991) Science 254:1375-1377). Deletion of Leu 2 (L2-) according to the invention causes the first N-terminal residue to be a serine (Ser3), which improves the half life of L-arabinose isomerase from *Geobacillus stearothermophilus* from about 2 minutes to an estimated half-life of more than 10 hours (estimated using the online protein analysis tool ProtParam: http ://web.expasy.org/protparam/; March 2012).

### Nucleic acid molecule

In a second aspect, the invention relates to a nucleic acid molecule encoding for a protein according to the invention as e.g. defined herein above.

A preferred nucleic acid molecule encoding a protein according to the invention is represented by:
i. nucleotide sequences encoding a protein, said protein comprising an amino acid sequence that has at least 95%, 96%, 97%, 98%, or 99% sequence identity with an amino acid sequence of any one of SEQ ID NO: 3 or SEQ ID NO: 4;
ii. nucleotide sequences comprising a nucleotide sequence that has at least 85%, 86%, 87%, 88%, 89%, 90%, 91%, 92%, 93%, 94%, 95%, 96%, 97%, 98%, or 99% sequence identity with a nucleotide sequence of any one of SEQ ID NO: 5 - 7;
iii. nucleotide sequences the complementary strand of which hybridizes to a nucleic acid molecule of sequence of (i) or (ii);
iv. nucleotide sequences the sequences of which differs from the sequence of a nucleic acid molecule of (iii) due to the degeneracy of the genetic code.

A further preferred nucleic acid molecule encodes a protein according to the invention having one or more of the conservative and/or preferred amino acid substitutions as defined herein above.

In a preferred embodiment, a nucleic acid molecule encoding a protein is represented by at least 85%, 86%, 87%, 88%, 89%, 90%, 91%, 92%, 93%, 94%, 95%, 96%, 97%, 98%, or 99%, most preferably 100% sequence identity with any one of SEQ ID NO: 5 - 7,. More preferably, a nucleic acid molecule encoding a protein or a part thereof is represented by any one of SEQ ID NO: 5 - 7.

A nucleic acid molecule may be a RNA or DNA molecule and may be comprised in a vector. The vector may be any (recombinant) DNA or RNA vector known in the art, and preferably is a plasmid; wherein genes encoding a protein or a fragment thereof are operably linked to regulatory sequences conferring expression and translation of the encoded messengers.

### Nucleic acid construct

In a third aspect, the invention relates to a nucleic acid construct, comprising a nucleic acid molecule according to the invention operably linked to a promoter for expression in a host cell.

A preferred nucleic acid according to the invention is a nucleic acid construct, wherein the nucleotide sequence encoding a protein of the invention is operably linked to a promoter and optionally other regulatory elements such as e.g. terminators, enhancers, polyadenylation signals, signal sequences for secretion and the like. The promoter and regulatory elements are preferably functional in the host cell for expression of the protein of the invention, such as the host cells described herein below. Such nucleic acid constructs are particularly useful for the production of a protein of the invention using recombinant techniques in which a nucleotide sequence encoding the protein of the invention is expressed in suitable host cells such as described in Ausubel et al., "Current Protocols in Molecular Biology", Greene Publishing and Wiley-Interscience, New York (1987) and in Sambrook and Russell (2001) "Molecular Cloning: A Laboratory Manual (3rd edition), Cold Spring Harbor Laboratory, Cold Spring Harbor Laboratory Press, New York). As used herein, the term "operably linked" refers to a linkage of polynucleotide elements in a functional relationship. A nucleic acid is "operably linked" when it is placed into a functional relationship with another nucleic acid molecule. For instance, a promoter or enhancer is operably linked to a coding sequence if it affects the transcription of the coding sequence. Operably linked means that the DNA sequences being linked are typically contiguous and, where necessary to join two protein coding regions, contiguous and in reading frame.

### Host cell

In a fourth aspect, the present invention pertains to a host cell comprising a nucleic acid molecule or a nucleic acid construct according to the invention as defined above. Preferably, the host cell is a host cell for production of a protein of the invention.

The host cell may be any host cell capable of producing a protein of the invention, including e.g. a cell of an eukaryotic microorganism such as yeasts and filamentous fungi or a (cultured) mammalian, plant, insect, fungal or yeast host cell, including e.g. CHO-cells, BHK-cells, human cell lines (including HeLa, COS and PER.C6), Sf9 cells and Sf+ cells. A preferred host cell for production of a protein of the invention is however a prokaryotic host cell. Various prokaryotic host cells may be utilized within the context of the present invention. Generally, preferred prokaryotic host cells should have a well-characterized genetic system, including known cloning vectors and methods of genetic manipulation. They should also preferably grow well in minimal medium, ideally to a high cell density, and without any special requirements (physical or physiological). Representative examples of such host cells include members of the *Bacillaceae*, *Nocardiaceae*, *Streptomycetaceae*, *Pseudomonadaceae*, *Corynebacteria*, and *Enterobacteriaceae*. Preferred host cells in the Family *Enterobacteriaceae* include *Escherichia*, *Citrobacter*, *Klebsiella*, *Enterobacter*, and *Serratia*, as well as *Zymomonas* and *Flavobacterium*, which are within the *Enterobacteriaceae* but of uncertain affiliation. Particularly preferred host cells include *Escherichia coli*, *Klebsiella oxytoca*, and *Klebsiella aerogenes*. In a preferred embodiment, the host cell is *E. coli* strain BL21 as described by (Studier and Moffatt, 1986, J. Mol. Biol. 189:113-130) as publicly available from e.g. the CBS (Centraalbureau voor Schimmelcultures, Utrecht, The Netherlands) under accession no. NCCB Nr 2889.

The above-described prokaryotes may be readily obtained from a variety of commercial sources including, for example, the American Type Culture Collection (ATCC) (Rockville, Md.). Alternatively, many of the above-described bacteria may be isolated from sources which are known by those of skill in the art to contain such prokaryotes, based upon techniques which are known in the art (see Bergy's Shorter Manual of Determinative Bacteriology, Williams & Wilkins (pub.), John G. Holt (ed.), 8th edition, 1977).

### Method for the production of a protein of the invention

In a fifth aspect, the invention relates to a method for the production of a protein of the invention, wherein the method preferably comprises the steps of: a) culturing a host cell as earlier defined herein under conditions conducive of producing the protein; and b) optionally isolating the protein. The protein may be isolated from at least one of the host cell and the culture medium.

Preferably, the culture medium used to propagate cells for the production of a protein according to the invention is maintained at a controlled pH for enhanced production of the isomerase and optimum enzymatic activity. A pH from 4 to 9 should preferably be maintained during propagation, preferably a pH from 5.5 to 7.

The culture medium used to propagate cells for the production of the protein of the present invention is preferably maintained at a temperature of 30 - 80°C, more preferably 30 - 70°C, 35 - 65°C, 35 - 60°C, 35 - 55°C or 35 - 50°C.

Suitable conditions may include the use of a suitable medium, the presence of a suitable source of food and/or suitable nutrients, a suitable temperature, and optionally the presence of a suitable inducing factor or compound (e.g. when the nucleotide sequences of the invention are under the control of an inducible promoter); all of which may be selected by the skilled person. For example, production of a protein of the invention may be performed in standard bioreactors with a working volume between 10 and 10,000 litres. Dissolved oxygen can be controlled by automatic adjustment of the impeller speed. The pH can be controlled using phosphoric acid and ammoniac gas or ammonia solution and temperature controlled via e.g. a cooling jacket and heating jacket.

A protein of the invention may be expressed in a constitutive manner, in a transient manner, or only when suitably induced. The proteins of the invention may then be isolated from the host cell/host organism and/or from the medium in which said host cell or host organism was cultivated, using protein isolation and/or purification techniques known per se, such as (preparative) chromatography and/or electrophoresis techniques, differential precipitation techniques, affinity techniques (e.g. using a specific, cleavable amino acid sequence fused with the amino acid sequence of the invention) and/or preparative immunological techniques (i.e. using antibodies against the protein to be isolated).

### Method for the production of alginate beads

In a sixth aspect, the present invention pertains to a method for the production of alginate beads comprising a protein according to the invention, wherein the method comprises the steps of: a) culturing a host cell as defined above under conditions conducive of producing a protein according to the invention to obtain a cell culture comprising the host cell that comprises the protein of the invention; b) adding 0.5 - 10 % (w/v) alginate to the cell culture obtained in step a) and incubate for 1 - 10 days at 45 - 100°C c) adding the mixture obtained in step b) dropwise into a solution comprising divalent cations, to obtain beads with a mean diameter of 0.5 to 3 mm; d) incubating the solution comprising the beads obtained in step c) at 1 - 10 °C for 1 - 36 hours harden the beads; and, e) isolating the hardened beads obtained in step d), preferably by filtration and/or centrifugation.

In a preferred embodiment, the cell culture period in step a) is for at least 1, 2, 3, 4, 5, 6, 7, 8, 9, 10 days. In a preferred embodiment this is done in a batch fermentation system and the preferred production takes 1 day. If the culture period in step a) is 2-3 days, it may result in more active enzyme, but in an industrial production, it is usually not advantageous to ferment for several days. If the culture period is 5 days or more, the possibility of cell lysis and contamination as well as loss of plasmid by the cells increases and is therefore less preferred. Moreover, when the enzyme is aging, the time to reach a specific galactose conversion (e.g., 30%) is longer.

Alginate is a carbohydrate extracted from seaweed and is preferably added to a final concentration of 0.5 - 10 % (w/v), more preferably 1 - 9, 1.5 - 7, 2 - 5 % (w/v). More preferably, alginate is added in step b) in the form of alginate powder and in a concentration of 2% W/V.

In a preferred embodiment, the incubation in step b) with alginate is carried out for 1 -24 hours, more preferably 3 - 20 hours, more preferably 8 - 18 hours, 10 - 17 hours, 14-17 hours, most preferably overnight. The incubation temperature in step b) preferably is 45 - 100°C, more preferably 50 - 90, 55 - 80, 60 - 75, 60 - 70, 60 - 65°C. Most preferably, the incubation temperature is 60°C, because it provides the best balance between the alginate solubility and AI stability.

Alternatively or in combination with other embodiments of the invention, in an embodiment of the invention, the dropwise addition of the mixture of b) (extrusion-dripping) into a solution comprising divalent cations can be carried out by using a device such as for example a needle fitted on a syringe or a multi-channel manifold with 10 µl to 1 ml tips. Preferably, a multi-channel manifold with 1 ml tips is used since it gives 2-3 mm beads. 3 mm is probably the largest size limit. Smaller sizes are supposed to increase the rate of reaction by increasing the substrate (galactose) diffusion. But, since we have tested beads from 0.5 to 3 mm, without any differences on activity, the beads size is not limiting the diffusion of the substrate.

Examples of divalent cations are calcium, magnesium and barium. Preferably, the divalent cations are calcium, more preferably the solution is a calcium chloride solution, for example a 0.05 - 2 M, 0.1 - 1.0 M, 0.15 - 0.7 M, most preferably a 0.2 M CaCl₂ solution.

The technique of producing alginate beads is well-known in the art, see for example Chan et al. (2009) Journal of Colloid and Interface Science 338:63-72.

When the beads are produced at a high flow rate (e.g., 0.5L/h for 96 tips), gentle propeller stirring (e.g., 200 rpm) of the solution comprising divalent cations is necessary, because the beads are formed in the presence of Ca²⁺ and Ca²⁺ is chelated by the alginate on carboxylic groups. In a preferred embodiment, after the production in step c), the alginate beads in the divalent cation bath are at least 1, 2, 3, 4, 5, 6, 7, 8, 9, 10, 11, 12 h, preferably less than 48, 36, 30, 26, 24, 20, 18, 16, 15, 14, 13 h incubated at 1 - 10 °C to harden the beads. The temperature at which the beads are incubated preferably ranges from 1- 10, more preferably from 2 - 9 , 3 - 8, 3 - 7, 3 - 5°C. It is most preferred that the temperature at which the beads are incubated is 4°C.

After isolating the beads in step d), the beads are preferably stored in galactose 350g/l, 50mM Tris, 1mM MnCl₂, pH=7.5. Preferably the beads are stored at 4°C. They can be stored in that solution for at least 3 months at 4°C without significant loss of activity.

### Solid support comprising a protein of the invention

In a further aspect, the invention relates to a composition comprising a protein as defined herein. A preferred embodiment thereof is a solid support comprising a protein of the invention. The solid support may be any material that may be used to for immobilization of a protein. Suitable examples are matrix materials, to entrap the protein, cell surfaces on which the protein is displayed and polymers that can be covalently linked to the protein. Examples of suitable solid supports are e.g., porous solid phase carrier materials such as agarose, polystyrene, controlled pore glass, cellulose, dextrans, kieselguhr, synthetic polymers such as Sepharose™, porous amorphous silica. In a preferred embodiment the solid support material is alginate, more preferably calcium alginate. The solid support materials may be in any suitable format such as particles, powders, sheets, beads, filters and the like, most preferably in the form of beads, such as alginate beads. In a preferred embodiment, the protein of the invention is entrapped in beads with a mean diameter of 2 - 3 mm, most preferably calcium alginate beads with a mean diameter of 2 - 3 mm.

Methods are available for immobilizing proteins quickly, easily and safely through a chosen functional group. The correct choice of coupling method depends on the substance to immobilized. For example the following commercially known derivatives of Sepharose™ allow the convenient immobilization of proteins thereon: CNBr-activated Sepharose™ 4B enables proteins containing primary amino groups to be rapidly immobilized by a spontaneous reaction. AH-Sepharose™ 4B and CH-Sepharose™ 4B both have a six-carbon long spacer arm and permit coupling via carboxyl and amino groups, respectively. Flexible spacers are suitable for use in situations where the flexibility of the target molecules is limited or where 3-dimensional structure of the target requires some flexibility of the binding agent to allow optimal binding. Activated CH-Sepharose™ 4B provides a six-carbon spacer arm and an active ester for spontaneous coupling via amino groups. These are only a few examples of suitable immobilisation routes. Optionally the solid support material comprising a protein of the invention is put into a column to facilitate easy conversion of galactose into tagatose.

Thus, in an aspect, the present invention relates to a solid support onto which a protein according to the invention is immobilized or within which a protein according to the invention is enclosed, i.e. within which the protein is immobilized. An advantage thereof is that the protein can be easily recovered to be used again. Another advantage is that it provides better stability, for example with respect to pH and temperature, it is cheap and it is more easy in industrial application.

In a preferred embodiment, the solid support is selected from the group consisting of: alginate, chitin, chitosan, cellusose, and silica. In a more preferred embodiment, the solid support is an alginate bead within which a protein according to the invention is trapped, *i.e.*, wherein the protein is immobilized.

In another aspect, the present invention pertains to an alginate bead comprising a protein of the invention and which is obtainable by the method defined above.

### Method for the production of D-tagatose

In a further aspect, the present invention relates to a method for the production of D-tagatose, the method comprising the steps of: i) incubating a sample comprising D-galactose with a protein according to the invention, a solid support as defined above or an alginate bead as defined above, under conditions conducive of production of D-tagatose; and ii) optionally at least one of purification and recovery of the D-tagatose.

A variety of samples comprising galactose can be used in a method for the production of D-tagatose. Galactose can be found in dairy products, sugar beets, and other gums and mucilages. Galactose can for example be obtained from milk or cheese or whey as described In US patent No. 6,057,135. For commercial production of D-tagatose, it is preferred to employ relatively concentrated D-galactose, at least 5% by weight, as starting material. A preferred range of D-galactose concentration in the starting material is from 10% to 60% by weight. In a preferred embodiment, a sample comprising galactose comprises, more preferably consists of, 350 g/L galactose, 50 mM Tris, 1 mM MnCl₂, pH 7.5. In a preferred embodiment, the galactose is produced from lactose from whey permeate.

The method for the production of D-tagatose is preferably carried out in the presence of metal ions, preferably divalent cations that are nutritionally safe or food grade. Productivity of the isomerase can for example be enhanced because isomerase require metal ions as cofactor. Suitable ions include manganese, magnesium, ferric, ferrous, cobalt, calcium and zinc, which are preferably used in the range of 0.01 to 5 mM, more preferably 0.1 - 2 mM and most preferable about 1 mM. The selected ion and optimum ion concentration for a given D-galactose isomerase will vary based on the microorganism used as source of D-galactose isomerase. When D-tagatose is being produced for food application, however, certain ions such as cobalt are preferably avoided. In an embodiment, 1 mM Mn²⁺ is used.

In a preferred embodiment, the incubation of the method for the production of D-tagatose is carried out at a pH from 5.0 to 8.0. More preferably at a pH from 7.5 - 8.0 since acidic pH impairs the stability of GSAI. More preferably, the incubation is carried out in a culture medium comprising a buffer, such as for example a Tris-HCl buffer.

The protein employed according to the invention can be used in whole-cell, cell free or immobilized systems. Effective isomerisation to produce D-tagatose from D-galactose can be conducted in a continuous, semi-continuous or batch operation, consistent with the enzyme system employed. In a preferred embodiment, beads (e.g. alginate beads) comprising a protein according to the invention are packed into a column, such as for example a XK16 column (GE Healthcare), on-column conversion of D-galactose into D-tagatose is performed.

In a preferred embodiment, the incubation is carried out at a temperature within the range of 40 - 100°C, preferably within the range of 50 - 70°C, more preferably at 60°C.

If applicable, the sample can be stirred during incubation, for example at a speed of 400-800 rpm. If conversion takes place within a column a flow rate of 0.5 - 10 mL per hour, preferably 1 - 9, 2 - 7, 2.5 - 6, 3 - 5, or 3 - 4 mL per hour is preferably applied.

In a preferred embodiment, the method further comprises the steps of purifying or isolating the tagatose using one or more methods selected from the group consisting of passing over an active carbon column, cation exchange chromatography, SMB, crystalisation, and drying.

### Use

In another aspect, the present invention relates to use of a protein according to the invention, a solid support as defined above or an alginate bead as defined above, in the enzymatic conversion of galactose into tagatose. The present invention also relates to use of the obtained tagatose in a food application. Therefore, the present invention also pertains to a method for the production of a food product, comprising the steps of the method for the production of D-tagatose as defined above and subsequently incorporating the D-tagatose in a food product.

Bacteria, e.g. E. coli, more specifically E. coli BL21, that are used for production of a protein according to the invention, must be eliminated before tagatose can be used in food. This requires purification of the tagatose using one or more of the following methods: passing over an active carbon column, cation exchange chromatography to remove metal ions such e.g. Mn2+, SMB for sugar separation and crystallisation to get a solid final product. Purification preferably includes passing over an active carbon column. Other purification steps will depend on the grade of purification that is required.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The following examples are offered for illustrative purposes only, and are not intended to limit the scope of the present invention in any way.

### Description of the figures

Figure 1. Schematic representation of the pCm470 GST vector for the expression of AI from Geobacillus stearothermophilus (GST).
Figure 2. Schematic representation of the pNUT005 vector for the expression of the NUT5 mutant of AI from *Geobacillus stearothermophilus* (SEQ ID NO: 8).
Figure 3. Analysis of G. *stearothermophilus* galactose isomerase (GSGI) production in E. coli strain BL21 using proteins from whole culture after 24h of fermentation using SDS-PAGE gel electrophoresis.
Figure 4. Standard curve for determining AI activity using a concentration range of tagatose. The amount of tagatose (ketose) is a measure for the D-galactose isomerase activity and is determined with a spectrophotometer at a wavelength of 560 nm after a colour forming of ketoses with cysteine / carbazole / sulphuric acid reagent.

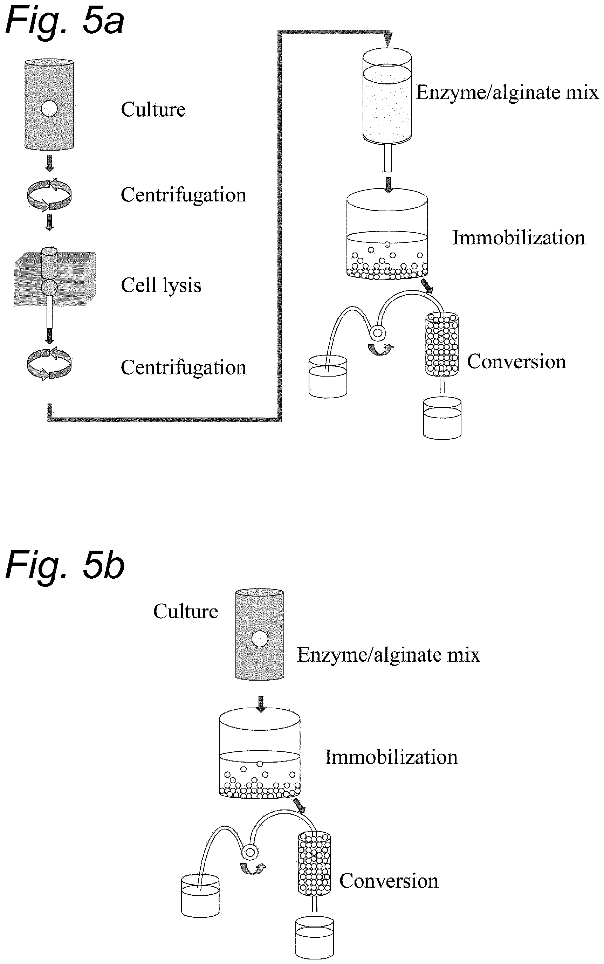

Figure 5. Schematic representation of the standard process of encapsulation of NUT10, NUT11, NUT13 and WT GSAI (panel A) and an improved process of encapsulation of proteins (panel B). Panel A : After fermentation, the enzyme is collected in the pellet after centrifugation. Then, the cells are lysed and the cell debris collected by a second centrifugation step. The supernatant of the second centrifugation step is mixed with a 4% alginate solution to allow for the production of alginate beads in which the proteins are encapsulated. Panel B : The lysis step and two centrifugation steps of panel A are omitted. Alginate is added directly to the cell culture, at the end of the incubation period and the beads are directly produced from the fermenter broth.
Figure 6. Activity of D-galactose isomerase from *Geobacillus stearothermophilus* (WT) and mutants (pNUT13, pNUT11 and pNUT10) after encapsulation in alginate beads and tested in a XK16/30 column. The figure shows the rate of galactose conversion after 48 hours at 60°C.

**Sequence listing**

| **SEQ ID NO.** | **description** | **type sequence** |
|---|---|---|
| 1 | L-arabinose isomerase wild-type (G. stearothermophilus) | amino acid |
| 2 | pNUT10 | amino acid |
| 3 | pNUT11 | amino acid |
| 4 | pNUT13 | amino acid |
| 5 | pNUT10 | nucleic acid |
| 6 | pNUT11 | nucleic acid |
| 7 | pNUT13 | nucleic acid |
| 8 | pNUT5 | amino acid |

### Examples

### Example 1. Design of mutants of GSAI to improve enzyme stability and activity at high temperature

### Example 1.1

D-galactose isomerase from *Geobacillus stearothermophilus* (SEQ ID NO: 1; Accession no.: Q9S467; encoded by the *G. stearothermophilus araA* gene) was selectively mutated at positions 32, 33, 64, 80, 88, 140 and 208 to increase the stability and activity of the enzymes in mainly limiting part of the potential oxidation sites. In addition, the leucine residue at position 2 was deleted in order to improve the cellular half life of the GSAI.

The methionine residues at positions 32 and 88 of SEQ ID NO:1 have been mutated to serine residues, the methionine residue at position 33 of SEQ ID NO:1 has been mutated to a lysine residue, the cysteine residue at position 64 of SEQ ID NO:1 has been mutated to phenylalanine, the methionine residues at positions 80 and 140 have been mutated to leucine residues, and the glycine residue at position 208 of SEQ ID NO:1 has been mutated to a threonine residue. This results in the sequence named NUT13 (depicted in SEQ ID NO:4). Thus, the following cumulated mutations/deletions were introduced into the sequence of D-galactose isomerase from G. *stearothermophilus*: L2-; M32S; M33K; C64F; M80L; M88S; M140L; G208T.

### Example 1.2

D-galactose isomerase from *Geobacillus stearothermophilus* (SEQ ID NO:1) was selectively mutated at positions 1, 32, 33, 64, 80, 88, 140, 208, 285, 297, 318 and 321 to increase the activity and the stability of the enzymes in mainly limiting the potential oxidation sites. The following mutations/deletions were introduced into the sequence of D-galactose isomerase from *G. stearothermophilus:* L2-; M32S; M33K; C64F; M80L; M88S; M140L; G208T; M285L; M297L; M318L; M321L, resulting in the sequence named NUT011 (SEQ ID NO:3).

### Example 1.3

D-galactose isomerase from *Geobacillus stearothermophilus* (SEQ ID NO:1) was selectively mutated at positions 345, 355, 380, 419, 435, 452, 468 and 473 to increase the activity and the stability of the enzymes in mainly limiting the potential oxidation sites. The following mutations were introduced: M343L; C353D; A377V; M416L; L432F; C449V; M465I; C470L, resulting in the sequence named NUT10 (SEQ ID NO:2).

The results of the mutations described in Examples 1.1, 1.2 and 1.3 are summarized in Table 1.

**Table 1. Description of the designed mutants.**

| name | mutations |
|---|---|
| WT* | |
| pNUT10 SEQ:2 | M343L; C353D; A377V; M416L; L432F; C449V; M465I; C470L |
| pNUT11 SEQ:3 | L2-; M32S; M33K; C64F; M80L; M88S; M140L; G208T; M285L; M297L; M318L; M321L |
| pNUT13 SEQ:4 | L2-; M32S; M33K; C64F; M80L; M88S; M140L; G208T |

| | |
|---|---|
| *D-galactose isomerase from *Geobacillus stearothermophilus* (SEQ ID NO:1) | |

### Example 2 Production of GSAI mutants

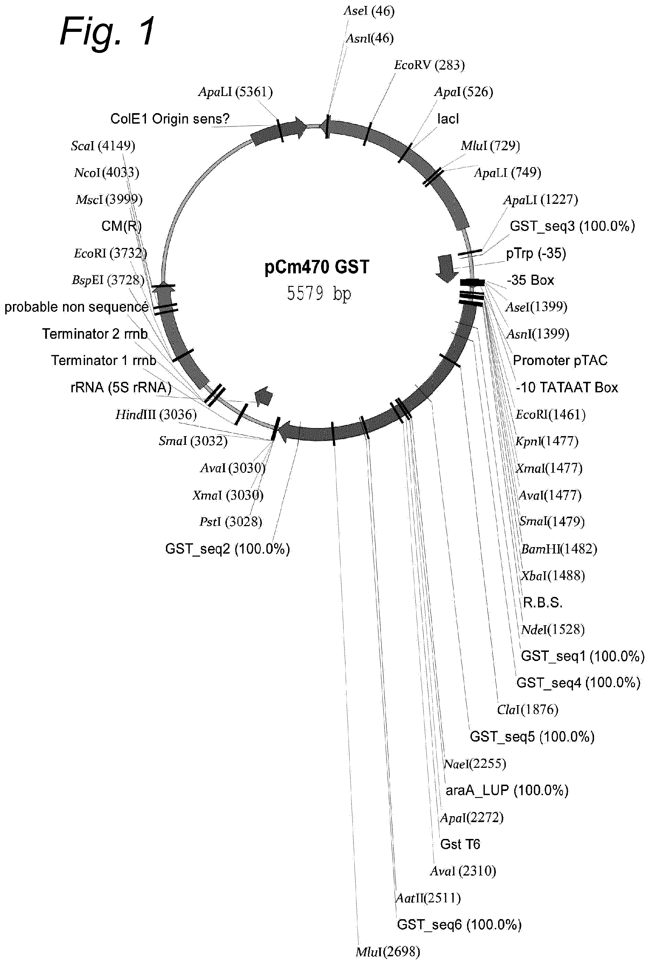

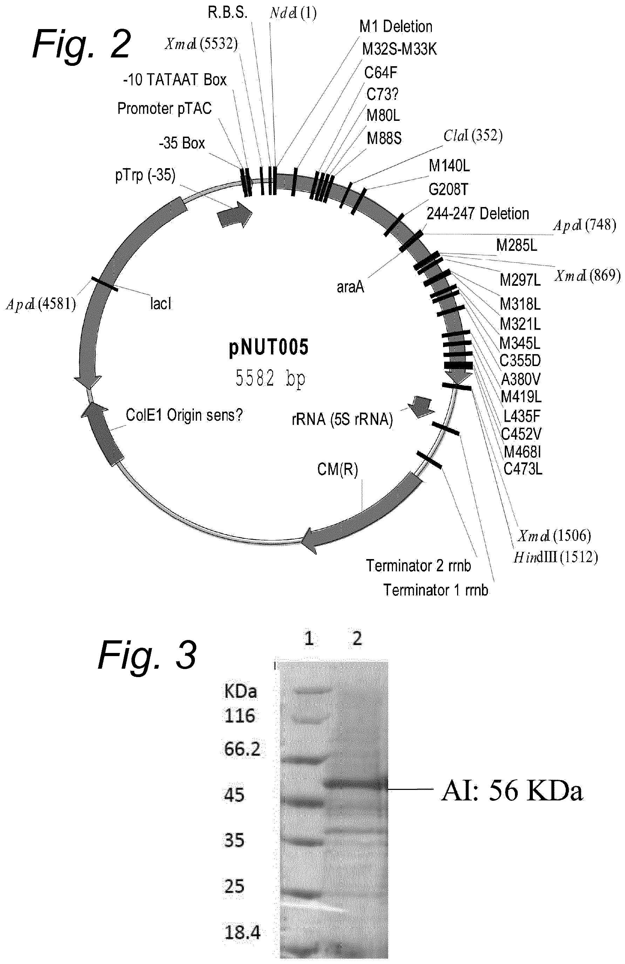

For production of D-galactose isomerase, the gene of L-arabinose isomerase from *Geobacillus stearothermophilus* has been introduced into a pCm470 vector and named pCm470 GST (Figure 1). pCm470 is a pUC19-based vector wherein the D-galactose isomerase coding sequences are for the pTac promoter. The pCm470 vector has been constructed using a pTac promoter based with NdeI - PstI/SmaI/ HindII cloning sites. The ribosome binding site (R.B.S.) is the same as the pET family vector. It has two STOP codons followed by a classical twofold rrnb terminator. pCm470 further comprises the lacI lac repressor gene and the chloramphenicol acetyltransferase (CAT) gene for selection of transformants based on chloramphenicol resistance. To produce the mutated AI proteins as described in Example 1.1 - 1.3, a single gene was synthesised, wherein the proposed mutations were included. The risk associated to this approach is the possibility that one or more deleterious mutations with respect to folding and/or activity are generated. In such a case, the deleterious mutation(s) can be rapidly identified (and reversed) by testing blocs of 3 to 4 mutations. To this effect, an artificial gene coding for SEQ ID NO:8, designed from D-galactose isomerase from Geobacillus stearothermophilus, was purchased (Genescript). This sequence incorporates all the mutations/deletions presented in example 1: L2-; M32S; M33K; C64F; M80L; M88S; M140L; G208T; M285L; M297L; M318L; M321L; M343L; C353D; A377V; M416L; L432F; C449V; M465I; C470L. To facilitate expression, the artificial gene has been introduced into the pCm470 vector (NUT005; Figure 2).

### Example 2.1. Construction of pNUT010, pNUT11 and pNUT13.

Hybrids between the wild type and mutated AI gene were built using restriction enzymes. For production of vector pNUT010 (incorporating mutations M343L; C353D; A377V; M416L; L432F; C449V; M465I; C470L) and vector pNUT011 (incorporating mutations/deletions L2-; M32S; M33K; C64F; M80L; M88S; M140L; G208T; M285L; M297L; M318L; M321L), pCm470 and pNUT005 were both digested using *Nde*I and *Aat*II*.* The appropriate fragments were combined to create the expression vector for NUT10 (SEQ ID NO:2) and NUT11 (SEQ ID NO:3). Similarly, for production of vector pNUT013 (incorporating mutations L2-; M32S; M33K; C64F; M80L; M88S; M140L; G208T), pCm470 and pNUT005 were both digested using *Apa*I. The appropriate fragments were combined to create the expression vector for NUT 13 (SEQ ID NO:4).

The *Escherichia coli* BL21 production strain (GE Healthcare) is used as expression system together with plasmids where an inducible pTac promotor is incorporated. This system gives a high expression of the D-galactose isomerase enzyme and therefore is suitable for a commercial use.

The vectors pNUT010, pNUT011, pNT013, comprising the nucleic acid sequences encoding for the polypeptides NUT10, NUT11 and NUT 13, respectively, were used to transform *E. coli* strain BL21 using classical molecular biology protocols. Cryostocks were prepared from the transformed strains to be used in further productions in a 15 L fermenter.

### Example 2.2. Production of pNUT010 in 15L fermenter

The day before fermentation, 5 cultures of 3 ml of BL21 strain transformed with pNUT010, pNUT011 or pNUT013, respectively (5 cultures per plasmid), were grown during 4 hours at 37°C in 2XYT medium (16 g/L tryptone; 10 g/L yeast extract; 5 g/L NaCl; 0.197 g/L MnCl₂.4H₂O) supplemented with chloramphenicol (12.5 µg/ml final concentration). Subsequently, the cultures were transferred to a larger volume of 50 ml during 2 hours at 37°C. The cultures were then placed in 1 L of 2XYT medium and incubated at 37°C over-night (at 250 rpm). A ratio of 1/50 (i.e. approx. 300 mL) was used for the inoculation of the 15 L fermenter. The preculture was added under sterile conditions to the fermenter containing 15 L of 2XYT medium, 12.5 µg/ml of chloramphenicol and 1 mM of MnCl₂.4H₂O. The culture was incubated at 37°C until the optical density at 600 nm reached a value of 2.0. Then, the production of NUT10, NUT11 or NUT 13, respectively, was induced with a final concentration of 2 mM IPTG and a 2% glucose fed-batch alimentation to the fermenter was started. The culture temperature was lowered to 25°C during the fed-batch phase. The pH was regulated at 7.0 with acid (H₃PO₄ 4 M) or base (NaOH 4 M) using a computer driven pump system. The percentage of oxygen was also regulated by computer. After 24h, the biomass was collected of the approx. 15 L culture by centrifugation: 17000 g for 15 min. For the E. coli transformed with pNUT010, 320 g of pellet was re-suspended in 320 ml of Tris buffer 50 mM, pH 7.5. The optical density at 600 nm of the culture in the fermenter reached a value of 22 after 24 hours. For the E. coli transformed with pNUT011, 378 g of pellet was re-suspended in 378 ml of Tris buffer 50 mM, pH 7.5. The OD₆₀₀ of the culture in the fermenter reached a value of 24 after 24 hours. For the E. coli transformed with pNUT013, 247 g of pellet was re-suspended in 247 ml of Tris buffer 50 mM, pH 7.5. The OD₆₀₀ of the culture in the fermenter reached a value of 24 after 24 hours.

These data are summarized in table 2 and compared to a production of GSAI performed under the same conditions. Figure 3 shows the protein composition of the total culture after 24 hours using SDS-PAGE gel electrophoresis. The protein corresponding to GSAI (56 Kda) has the most intense band.

**Table 2. Comparison of biomass produced upon culture of NUT10, NUT11, NUT 13 and WT GSAI in 15 L fermenter.**

| **Name** | **Wet biomass (g)** | **OD₆₀₀** |
|---|---|---|
| WT | 450 | 26 |
| NUT10 | 320 | 22 |
| NUT11 | 378 | 24 |
| NUT13 | 247 | 17 |

### Example 3. Determination of galactose conversion activity in cell culture, pellet and supernatant.

### Materials and Methods

After 24 hours of fermentation (Example 2), 1 ml samples from the cultures (NUT10, NUT11 and NUT13; WT Geobacillus stearothermophilus D-galactose isomerase as a control) were taken for determination of galactose conversion activity. To measure the activity in the pellet, the biomass is first concentrated by centrifugation (15 min at 17000 g). The mass of the pellet was determined and subsequently, the pellet was resuspended in a volume of 50 mM Tris pH 7.5 buffer 2x the initial volume of the pellet (e.g., 450 g of pellet was resuspended in 900 ml of buffer for WT) to obtain 50% (w/v) resuspended pellet. Then, 20 µl of the resuspended pellet was added to 180 µl of 385 g/L galactose.

The supernatant resulting from the centrifugation step was also assayed for galactose conversion activity. For cultures and supernatants, 100 µl of sample was added to 100 µl of 700g/L galactose (heated at 60°C for 60 min). Tris and MnCl₂ were added to the galactose solution to reach final concentrations of 1 mM MnCl₂ and 50mM Tris (pH 7.5).

The samples were incubated for 1 hour at 60°C. One unit is herein defined as the amount of protein that is required to produce 1 gram of D-tagatose per liter from D-galactose following incubation for 1 hour at 60°C under assay conditions.

The method that was used for tagatose detection can be applied for the determination of relative D-galactose isomerase activity in fermentation, downstream processing steps and immobilized samples. The protocol is based on the method of Dische and Borenfreund [J.Biol.Chem. 192: 583-587 (1951)].

D-galactose isomerase catalyzes the isomerisation of arabinose into ribulose (L-arabinose isomerase). Due to the strong structural resemblance, galactose molecules do fit most likely into the active site as well, allowing D-galactose isomerase to isomerise D-galactose, but at much lower rate and with a much lower affinity (higher Km) than the isomerisation of arabinose into ribulose. The conversion of galactose into tagatose is highly specific, no other sugars are produced. The amount of tagatose (ketose) is a measure for the D-galactose isomerase activity and is determined with a spectrophotometer at a wavelength of 560 nm after a colour forming of ketoses with a cysteine / carbazole / sulphuric acid reagent as described in reference Dische and Borenfreund (1951) (100 µl of sample is transferred from the reaction sample tube to the microtitreplate. 100 µl of reagent is added to the microtitreplate and the incubation with the reagent is 30 min at 25°C. The test is performed in 96-wells microtitreplate in a final volume of 200µl. This method is a relative method. The results are related to a tagatose standard (Figure 4).

### Results

After downstream processing (DSP) and resuspension of the pellet, NUT10, NUT11 and NUT 13 were tested for D-galactose conversion into D-tagatose, using the above described method. The results are shown in Table 3.

For NUT13 an activity of 71% in comparison to the non-mutated D-galactose isomerase enzyme from *Geobacillus stearothermophilus* (WT) was determined. 96% of the AI activity of NUT 13 was found in the pellet. For WT GSAI, 55% of the D-galactose isomerase activity of the culture was found in the pellet.

For NUT11 an activity increment of 58% in comparison to the WT D-galactose isomerase enzyme from *G. stearothermophilus* was determined. 53 % of the D-galactose isomerase activity of NUT11 was found in the pellet. For NUT10 an activity increment of 29% in comparison to the WT D-galactose isomerase enzyme from G. *stearothermophilus* was determined. 47 % of the D-galactose isomerase activity of NUT10 was found in the pellet.

**Table 3. Comparison of galactose conversion activities of NUT10, NUT11 and NUT13 with D-galactose isomerase from Geobacillus stearothermophilus after production in 15 L fermenter.**

| **Name** | **Culture** | **Supernatant** | **Pellet** | | | | |
|---|---|---|---|---|---|---|---|
| | *Activity (U)* | *Activity (U)* | *Activity (U)* | *Activity (% total)* | *mass (g)* | *Specific activity (U*/*g biomass)* | *Specific activity (%WT)* |
| WT | 1950 | 600 | 1080 | 55 | 450 | 2.4 | 100 |
| pNUT10 | 2100 | 600 | 992 | 47 | 320 | 3.1 | 129 |
| pNUT11 | 2700 | 1050 | 1436 | 53 | 378 | 3.8 | 158 |
| pNUT13 | 1050 | 0 | 1013 | 96 | 247 | 4.1 | 171 |

### Example 4. AI enzyme immobilization and galactose conversion.

Alginate beads are known to increase enzyme stability. It is also a cheap way of enzyme immobilization and it is easily scaled up to industrial production. Alginate is appropriate for encapsulation of large polypeptides and minimal leaching is observed with GSAI, NUT10, NUT11 and NUT 13 where the functional unit is a tetramer of 220 kDa. It is also suitable for whole bacterial cell immobilization. Alginate encapsulation of AI enzymes is well described in the scientific literature (Zhang YW et al. (2010) Prep Biochem Biotechnol. 40(1):65-75; Zhang YW et al. (2010) Bioprocess Biosyst Eng. 33(6):741-748).

### Example 4.1. Process improvement.

Typically, 500 mL of the cells from the wet biomasses of the WT control, NUT10, NUT11 and NUT13 collected by centrifugation as described in Example 2 and re-suspended in one volume of 50 mM Tris pH 7.5 buffer are lysed using a cell disrupter (Panda NS1001L; GEA Liquid Processing; Niro Soavi PHARMA Laboratory Homogenizer). Subsequently, the soluble enzyme fraction is collected by centrifugation and 500 ml of a 4% alginate solution is added to the soluble enzyme fraction for the production of beads (panel A). Finally, the beads are separated from the fluid. However, in order to simplify the downstream processing described in panel A of figure 5 the whole cells expressing D-galactose isomerase of Geobacillus stearothermophilus (GSGI), NUT10, NUT 11, and NUT 13 were used rather than the soluble fraction resulting from the cell lysis, thereby thus leaving out the cell lysis step and two centrifugation steps. An additional level of simplification was introduced by adding 2 % (w/w) alginate powder (300 g/15L of fermentation medium = 300g/450 g of wet biomass) at the end of the 24 hour incubation directly to the cell culture, without collecting the cells from the biomass and resuspending them. The advantage of adding alginate as a powder instead of a solution, is that otherwise the biomass would be diluted and beads are obtained with lower activity. The beads were directly produced from the fermenter broth. The improved process thus consists in a fermentation of GSGI, NUT10, NUT11, and NUT 13 where the alginate powder (2% W/V) is added at the end of the 24H culture (figure 5, panel B). After stirring the alginate/fermenter broth overnight in the fermenter raised to 60°C, the beads are directly produced by pumping the alginate/cells mix from the fermenter to a 0.2 M CaCl₂ solution through a drop making multi-channel manifold which produces beads of 2 to 3 mm in diameter. When the beads are produced at a high flow rate, gentle propeller stirring of the calcium chloride solution is necessary. After the production, the beads in the CaCl₂ bath are transferred to 4°C O/N to harden the beads. The following day, the beads are separated from the bath by filtration and stored at 4°C in galactose 350g/l, 50mM Tris, 1mM MnCl₂, pH=7.5. They can be stored in that solution for at least 3 months at 4°C without significant loss of activity. The enzyme content of the beads has been estimated at 5% (w/v) whereas, with the previous downstream processing (Figure 5, panel A) it was slightly higher (8%). However, in an industrial process, the advantage brought by the simplification of the downstream processing and beads production largely compensate for the slightly less active beads.

### Example 4.2. Tagatose production after enzyme encapsulation.

30 ml of beads prepared directly from the GSGI, NUT10, NUT11 and NUT13 fermentations were loaded on XK16 columns (GE Healthcare) maintained at 60°C. A test fluid (350 g/L galactose, 50mM Tris, 1mM MnCl₂, pH=7.5) is loaded at a flow rate of 3 ml/h. Samples of 1 ml are collected after 48 h. The tagatose content is determined using method described in example 3.

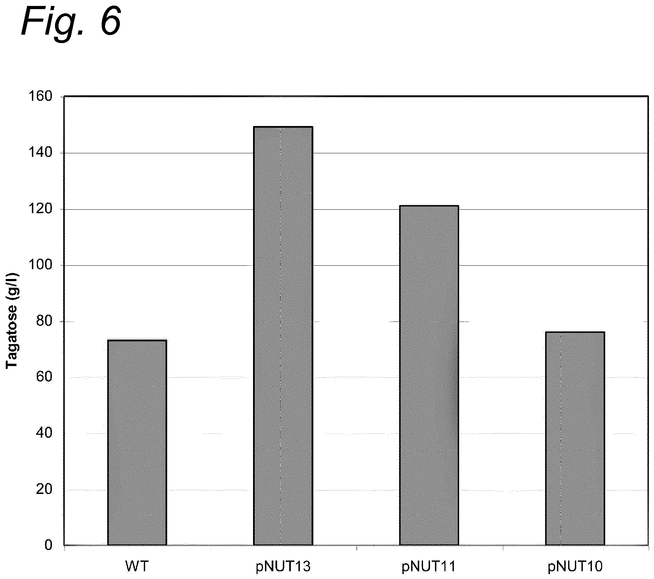

The results are shown in Figure 6. The NUT10 column shows the lowest improvement as compared to the WT control of GSGI (76 g/L vs. 73 g/L of tagatose for NUT10 and GSAI, respectively). For the NUT11 and NUT13 columns a much better galactose conversion activity was shown with a production of 121 and 149 g/L of tagatose respectively.

In conclusion, NUT10, NUT 11 and more specifically, NUT 13 once immobilized in alginate show rates of galactose conversion into tagatose compatible with an industrialized process. It is estimated that out of 10500 kg galactose more than 10 ton of tagatose can be produced per cubic meter of NUT 13 immobilized in alginate beads. When compared to D-galactose isomerase from *Geobacillus stearothermophilus*, encapsulated beads prepared with NUT 13 would require 51% less enzymatic material in an industrial production process of tagatose. For NUT11, 40% less enzymatic material is needed to achieve a similar amount of tagatose and for NUT10 4% less enzymatic material is needed to achieve a similar amount of tagatose as obtained in the control.

### References

Ansari S and Helms V 2005 Statistical analysis of predominantly transient protein-protein interfaces. Proteins 61, 344-355
Chouayekh H, Bejar W, Rhimi M, Jelleli K, Mseddi M, Bejar S 2007 Characterization of an L-arabinose isomerase from the Lactobacillus plantarum NC8 strain showing pronounced stability at acidic pH. FEMS Microbiol Lett. 277, 260-267
Delhaise PM 1984 Interactive computer animation of macromolecules. J. Mol. Graph. 2, 103-106
Desmet J, Spriet J, Lasters I 2002 Fast and accurate side-chain topology and energy refinement (FASTER) as a new method for protein structure optimization. Proteins 48, 31-43
Jorgensen F, Hansen OC, Stougaard P 2004 Enzymatic conversion of D-galactose to D-tagatose : heterologous expression and characterization of a thermostable L-arabinose isomerase from Thermoanaerobacter mathranii. Appl. Microbiol. Biotechnol. 64, 816-822
Kim HJ, Ryu SA, Kim P, Oh DK 2003 A feasible enzymatic process for D-tagatose production by an immobilized thermostable L-arabinose isomerase in a packed-bed bioreactor. Biotechnol. Prog. 19, 400-404
Kim HJ, Kim JH, Oh HJ, Oh DK 2006 Characterization of a mutated Geobacillus stearathermophilus L-arabinose isomerase that increases the production rate of D-tagatose. J. Appl. Microbiol. 101, 213-221
Lee DW, Jang HJ, Choe EA, Kim BC, Lee SJ, Kim SB, Hong YH, Pyun YR 2004 Characterization of a thermostable L-arabinose(D-galactose) isomerase from the hyperthermophilic eubacterium Thermotoga maritima. Appl. Environm. Microbiol. 70, 1397-1404
Lee DW, Choe EA, Kim SB, Eom SH, Hong YH, Lee SJ, Lee HS, Lee DY, Pyun YR 2005 Distinct metal dependence for catalytic and structural functions in the L-arabinose isomerases from the mesophilic Bacillus halodurans and the thermophilic Geobacillus stearothermophilus. Arch. Biochem. Biophys. 434, 333-343
Manjasetty BA, Chance MR 2006 Crystal structure of Escherichia coli L-arabinose isomerace (ECAI), the putative target of biological tagatose production. J. Mol. Biol. 360, 297-309
Prabhu P, Jeya M, Lee J 2010 Probing the molecular determinant for the catalytic efficiency of L-arabinose isomerase from Bacillus licheniformis. Applied Environm. Microbiol. 76, 1653-1660
Rhimi M, Juy M, Aghajari N, Haser R, Bejar S 2007 Probing the essential catalytic residues and substrate affinity in the thermoactive Bacillus stearothermophilus US 100 L-arabinose isomerase by site-directed mutagenesis. J. Bacteriol. 189, 3556-3563
Seemann JE, Schulz GE 1997 Structure and mechanism of L-fucose isomerase from Escherichia coli. J. Mol. Biol. 273, 256-268
Takeda K, Yoshida H, Izumori K, Kamitori S 2010 X-ray structure of Bacillus pallidus D-arabinose isomerase and its complex with L-fucitol. Biochem. Biophys. Acta. In press.
Thompson JD, Higgins DG, Gibson TJ 1994 CLUSTALW : improving the sensitivity of progressive multiple sequence alignment through sequence weighting, position-specific gap penalties and weight matrix choice. Nucleic Acids Res. 22, 4673-4680s.

## Claims

1. A protein comprising
a) an amino acid sequence of SEQ ID NO: 3 or SEQ ID NO: 4, or
b) an amino acid sequence with at least 95% amino acid identity with SEQ ID NO: 3 or SEQ ID NO: 4, or
c) an amino acid sequence which has at least 95% amino acid identity with SEQ ID NO: 1;
wherein the protein has D-galactose isomerase activity and, wherein the amino acid sequence comprises amino acid substitutions M32S, M33K, C64F, M80L, M88S, M140L and G208T and a deletion of L2.

2. A nucleic acid molecule encoding for the protein of claim 1.

3. A nucleic acid construct, comprising the nucleic acid molecule of claim 2 operably linked to a promoter for expression in a host cell.

4. A host cell comprising the nucleic acid construct of claim 3.

5. A host cell according to claim 4, wherein the host cell is *Escherichia coli*, preferably *E. coli* strain BL21.

6. A method for the production of a protein according to claim 1, the method comprising the steps of:
a) culturing a host cell of claim 4 or 5 under conditions conducive of producing the protein;
b) optionally isolating the protein.

7. A method for the production of alginate beads comprising a protein according to claim 1, wherein the method comprises the steps of;
a) culturing a host cell of claim 4 or 5 under conditions conducive of producing the protein according to claim 1 to obtain a cell culture comprising the host cell comprising a protein;
b) adding 0.5 - 10 % (w/v) alginate to the cell culture obtained in step a) and incubate for 1 - 24 hours at 45 - 100°C ;
c) adding the mixture obtained in step b) dropwise into a solution comprising divalent cations to obtain beads with a mean diameter of 0.5 to 3 mm;
d) incubating the solution comprising the beads obtained in step c) at 1 - 10 °C for 1 - 36 hours harden the beads;
e) isolating the hardened beads obtained in step d), preferably by filtration and/or centrifugation.

8. A solid support onto or within which a protein according to claim 1 is immobilized.

9. A solid support according to claim 8, wherein the solid support is selected from the group consisting of: alginate, chitin, chitosan, cellusose, and silica.

10. A solid support according to claim 9, wherein the solid support is an alginate bead within which a protein according to claim 1 is immobilized.

11. An alginate bead comprising a protein according to claim 1, obtainable by the method of claim 7.

12. A method for the production of D-tagatose, the method comprising the steps of:
i) incubating a sample comprising galactose with a protein according to claim 1, a solid support according to any one of claims 8 - 10 or an alginate bead according to claim 11, under conditions conducive of production of tagatose; and
ii) optionally at least one of purification and recovery of the tagatose.

13. A method according to claim 12, wherein the method further comprises the steps of purifying the tagatose using one or more methods selected from the group consisting of passing over an active carbon column, ion exchange chromatography, SMB, crystalisation and drying.

14. Use of a protein according to claim 1, a solid support according to any one of claims 8 - 10 or an alginate bead according to claim 11, in the enzymatic conversion of galactose into tagatose.

## Patentansprüche

1. Ein Protein, umfassend
a) eine Aminosäurensequenz mit der SEQ ID NO: 3 oder SEQ ID NO: 4, oder
b) eine Aminosäurensequenz mit mindestens 95 % Identität von Aminosäuren zur SEQ ID NO: 3 oder SEQ ID NO: 4, oder
c) eine Aminosäurensequenz mit mindestens 95 % Identität von Aminosäuren zur SEQ ID NO: 1;
wobei das Protein eine D-Galaktose-Isomerase-Aktivität aufweist und, wobei die Aminosäurensequenz die Aminosäurensubstitutionen M32S, M33K, C64F, M80L, M88S, M140L und G208T und eine Deletion von L2 umfasst.

2. Ein Nukleinsäuremolekül, welches für das Protein aus Anspruch 1 kodiert.

3. Ein Nukleinsäurekonstrukt, welches das Nukleinsäuremolekül aus Anspruch 2 umfasst, das operativ mit einem Promotor zur Expression in einer Wirtszelle verknüpft ist.

4. Eine Wirtszelle, welche das Nukleinsäurekonstrukt aus Anspruch 3 umfasst.

5. Eine Wirtszelle nach Anspruch 4, wobei die Wirtszelle *Escherichia coli*, vorzugsweise *E. coli* Stamm BL21, ist.

6. Ein Verfahren zur Produktion eines Proteins nach Anspruch 1, wobei das Verfahren die folgenden Schritte umfasst:
a) Kultivieren einer Wirtszelle nach Anspruch 4 oder 5 unter Bedingungen, die für die Produktion des Proteins förderlich sind;
b) wahlweise Isolieren des Proteins.

7. Ein Verfahren zur Produktion von Alginat-Kügelchen, die ein Protein nach Anspruch 1 umfassen, wobei das Verfahren die folgenden Schritte umfasst:
a) Kultivieren einer Wirtszelle nach Anspruch 4 oder 5 unter Bedingungen, die für die Produktion des Proteins nach Anspruch 1 förderlich sind, um eine Zellkultur zu gewinnen, welche die ein Protein enthaltende Wirtszelle umfasst;
b) Zugeben von 0,5 - 10 % (Gew./Vol.) an Alginat zur in Schritt a) erhaltenen Zellkultur und Inkubieren für 1 - 24 Stunden bei 45 - 100 °C;
c) tropfenweise Zugeben der in Schritt b) erhaltenen Mischung in eine Lösung, die zweiwertige Kationen enthält, um Kügelchen mit einem mittleren Durchmesser von 0,5 bis 3 mm zu gewinnen;
d) Inkubieren der Lösung, welche die in Schritt c) erhaltenen Kügelchen enthält, bei 1 - 10 °C für 1 - 36 Stunden zum Verfestigen der Kügelchen;
e) Isolieren der verfestigten, in Schritt d) erhaltenen Kügelchen, vorzugsweise mittels Filtration und/oder Zentrifugation.

8. Ein fester Träger, auf welchem oder in welchem ein Protein nach Anspruch 1 immobilisiert ist.

9. Ein fester Träger nach Anspruch 8, wobei der feste Träger ausgewählt ist aus der Gruppe bestehend aus: Alginat, Chitin, Chitosan, Cellulose und Kieselsäure.

10. Ein fester Träger nach Anspruch 9, wobei der feste Träger ein Alginat-Kügelchen ist, in welchem ein Protein nach Anspruch 1 immobilisiert ist.

11. Ein Alginat-Kügelchen, umfassend ein Protein nach Anspruch 1, das nach dem Verfahren nach Anspruch 7 erhältlich ist.

12. Ein Verfahren zur Produktion von D-Tagatose, wobei das Verfahren die folgenden Schritte umfasst:
i) Inkubieren einer Probe, die Galaktose enthält, mit einem Protein nach Anspruch 1, einem festen Träger nach einem der Ansprüche 8 - 10 oder einem Alginat-Kügelchen nach Anspruch 11, unter Bedingungen, die für die Produktion von Tagatose förderlich sind; und
ii) gegebenenfalls mindestens einen von Reinigung und Gewinnung der Tagatose.

13. Ein Verfahren nach Anspruch 12, wobei das Verfahren weiterhin die Schritte zum Reinigen der Tagatose umfasst, unter Verwendung von einem oder mehreren Verfahren ausgewählt aus der Gruppe bestehend aus Durchleiten über eine Aktivkohlesäule, Ionenaustauschchromatographie, SMB, Kristallisation und Trocknung.

14. Verwendung eines Proteins nach Anspruch 1, eines festen Trägers nach einem der Ansprüche 8 - 10 oder eines Alginat-Kügelchens nach Anspruch 11, bei der enzymatischen Umwandlung von Galaktose in Tagatose.

## Revendications

1. Protéine comprenant
a) une séquence d'acides aminés parmi SEQ ID NO:3 ou SEQ ID NO:4, ou
b) une séquence d'acides aminés avec au moins 95 % d'identité d'acides aminés avec SEQ ID NO:3 ou SEQ ID NO:4, ou
c) une séquence d'acides aminés qui a au moins 95 % d'identité d'acides aminés avec SEQ ID NO:1;
dans laquelle la protéine a une activité isomérase de D-galactose, et dans laquelle la séquence d'acides aminés présente des substitutions d'acides aminés M32S, M33K, C64F, M80L, M88S, M140L et G208T et une délétion de L2.

2. Molécule d'acide nucléique codant pour la protéine selon la revendication 1.

3. Construction d'acide nucléique, comprenant la molécule d'acide nucléique selon la revendication 2 liée de manière fonctionnelle à un promoteur d'expression dans une cellule hôte.

4. Cellule hôte comprenant la construction d'acide nucléique selon la revendication 3.

5. Cellule hôte selon la revendication 4, dans laquelle la cellule hôte est *Escherichia coli*, de préférence *E. coli* de souche BL21.

6. Méthode pour la production d'une protéine selon la revendication 1, la méthode comprenant les étapes consistant à :
a) mettre en culture une cellule hôte selon la revendication 4 ou 5 dans des conditions favorables à la production de la protéine;
b) facultativement isoler la protéine.

7. Méthode pour la production de perles d'alginate comprenant une protéine selon la revendication 1, dans laquelle la méthode comprend les étapes consistant à :
a) mettre en culture une cellule hôte selon la revendication 4 ou 5 dans des conditions favorables à la production de la protéine selon la revendication 1 pour obtenir une culture cellulaire comprenant la cellule hôte comprenant une protéine ;
b) ajouter 0,5 à 10 % (w/v) d'alginate à la culture cellulaire obtenue à l'étape a) et incuber pendant 1 à 24 heures à 45-100°C ;
c) ajouter le mélange obtenu à l'étape b) goutte à goutte dans une solution comprenant des cations divalents pour obtenir des perles ayant un diamètre moyen de 0,5 à 3 mm ;
d) incuber la solution comprenant les perles obtenues à l'étape c) à 1-10°C pendant 1-36 heures pour durcir les perles ;
e) isoler les perles durcies obtenues à l'étape d), de préférence par filtration et/ou centrifugation.

8. Support solide sur ou dans lequel une protéine selon la revendication 1 est immobilisée.

9. Support solide selon la revendication 8, dans lequel le support solide est choisi dans le groupe comprenant: alginate, chitine, chitosane, cellusose, et silice.

10. Support solide selon la revendication 9, dans lequel le support solide est une perle d'alginate à l'intérieur de laquelle une protéine selon la revendication 1 est immobilisée.

11. Perle d'alginate comprenant une protéine selon la revendication 1, pouvant être obtenue par la méthode de la revendication 7.

12. Méthode pour la production de D-tagatose, la méthode comprenant les étapes suivantes:
i) incubation d'un échantillon comprenant du galactose avec une protéine selon la revendication 1, un support solide selon l'une quelconque des revendications 8-10 ou une perle d'alginate selon la revendication 11, dans des conditions favorables de production de tagatose ; et
ii) éventuellement au moins parmi une purification et une récupération du tagatose.

13. Méthode selon la revendication 12, dans laquelle la méthode comprend en outre les étapes consistant à purifier le tagatose en utilisant une ou plusieurs méthodes choisies dans le groupe comprenant passage sur une colonne de charbon actif, chromatographie par échange d'ions, SMB, cristallisation et séchage.

14. Utilisation d'une protéine selon la revendication 1, d'un support solide selon l'une quelconque des revendications 8-10 ou d'une perle d'alginate selon la revendication 11, dans la conversion enzymatique de galactose en tagatose.
